(19) Europäisches Patentamt
European Patent Office
Office européen des brevets

(11) EP 2 213 672 A1

(12) **EUROPEAN PATENT APPLICATION**
published in accordance with Art. 153(4) EPC

(43) Date of publication:
**04.08.2010 Bulletin 2010/31**

(21) Application number: **08842644.0**

(22) Date of filing: **24.10.2008**

(51) Int Cl.:
***C07D 471/04*** *(2006.01)* ***A61K 49/00*** *(2006.01)*
***A61K 49/04*** *(2006.01)*

(86) International application number:
**PCT/JP2008/069335**

(87) International publication number:
**WO 2009/054496 (30.04.2009 Gazette 2009/18)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **24.10.2007 JP 2007276826**

(71) Applicant: **Nihon Medi-Physics Co., Ltd.**
**Tokyo 136-0075 (JP)**

(72) Inventors:
- **TANIFUJI, Shigeyuki**
  **Sodegaura-shi**
  **Chiba 299-0266 (JP)**
- **NAKAMURA, Daisaku**
  **Sodegaura-shi**
  **Chiba 299-0266 (JP)**
- **TAKASAKI, Shinya**
  **Sodegaura-shi**
  **Chiba 299-0266 (JP)**
- **OKUMURA, Yuki**
  **Sodegaura-shi**
  **Chiba 299-0266 (JP)**

(74) Representative: **Wilhelms · Kilian & Partner**
**Patentanwälte**
**Eduard-Schmid-Straße 2**
**81541 München (DE)**

# (54) NOVEL COMPOUND HAVING AFFINITY FOR AMYLOID

(57) A compound effective as a probe targeting amyloid for image diagnosis and a reagent comprising the compound for detecting amyloid deposited on a biological tissue are provided. Provided are a compound represented by the following formula (1):

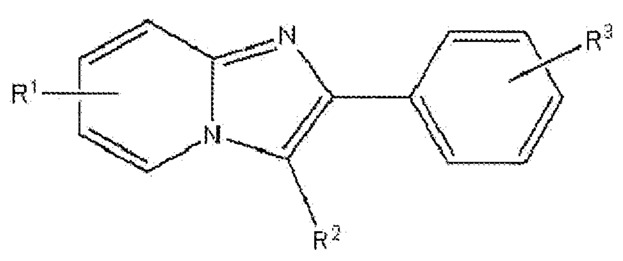

(1)

wherein $R^1$ is a group selected from the group consisting of a halogen substituent, alkyl substituent with 1 to 4 carbon atoms, alkoxy substituent having alkyl chain with 1 to 4 carbon atoms and hydroxyl group, $R^2$ is a group selected from the group consisting of a cyano substituent, alkyl substituent with 1 to 4 carbon atoms and halogen substituent, is a group selected from the group consisting of a hydroxyl group, halogen substituent and substituent represented by the following formula:

EP 2 213 672 A1

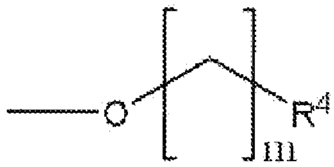

(wherein $R^4$ is a hydrogen, halogen substituent or hydroxyl group, and m is an integer of 1 to 4), provided that a t least one of $R^1$, $R^2$, $R^3$ and $R^4$ represents a radioactive halogen, and a reagent comprising the same.

## Description

### TECHNICAL FIELD

The present invention relates to a compound for use in diagnosis of cerebral degenerative disease. More specifically, the invention relates to a compound useful for amyloid detection at lesion sites in diagnosis of Alzheimer's disease and other diseases with amyloid accumulation.

### BACKGROUND ART

Diseases with the onset of deposition of a fibrous protein called amyloid in various organs or tissues in bodies are generally referred to as amyloidosis. A feature common to amyloidosis is that the fibrous protein called amyloid which is enriched with the β-sheet structure is deposited at various organs systemically or at sites topically so that functional abnormalities are triggered in the organs or tissues.

Alzheimer's disease (hereinafter referred to as AD), which is a typical amyloidosis disease, is known as a disease causing dementia. This disease is lethal with progressive deposition of amyloid in brain, and thus is said to be a disease that causes concern in society compared with other amyloidosis diseases. In recent years, the number of AD patients is rapidly increasing in developed countries with aging societies, thereby causing a social problem.

From the pathohistological viewpoint, AD is characterized by three pathological findings in brain, namely development of senile plaques, formation of neurofibrillary tangles, and extensive neuronal loses,

The senile plaque has a structure mainly composed of amyloid, and is said to appear at the earliest stage of AD onset and thus is pathologically found in brain about 10 or more years before appearance of clinical symptoms.

AD is diagnosed by carrying out various evaluations of cognitive functions (for example, Hasegawa scale, ADAS-JCog and MMSE) in auxiliary combination with imaging diagnosis such as CT and MRI. However, the method based on such evaluations of cognitive, functions is low in diagnostic sensitivity at the early stage of the onset, and is furthermore problematic in that diagnostic results are susceptible to inborn cognitive functions of individuals. At present, it is practically impossible to establish a definite diagnosis of AD while an AD patient is still alive, because the definite diagnosis requires a biopsy of a lesion (Non-Patent Document 1).

Meanwhile, a report tells that amyloid constituting senile plaques is an aggregate of amyloid β protein (hereinafter referred to as Aβ). Also, numerous resorts tell that the Aβ aggregate forms a β-sheet structure that causes nerve cell tonicity. Based on these findings, the so-called "Amyloid Cascade Hypothesis" is proposed, which suggests that cerebral deposition of Aβ triggers the downstream phenomena, namely, formation of neurofibrillary tangles and neuronal loss (Non-Patent Document 2).

Based on these facts, attempts have recently been made to detect AD *in vivo* using a compound having high affinity with amyloid as a marker.

Many of such probes for imaging diagnoses of cerebral amyloid are hydrophobic low-molecular weight compounds that are high in affinity with amyloid and high in cerebral transferability and are labeled with various radioactive species such as $^{11}C$, $^{18}F$ and $^{123}I$. For example, reports tell $^{11}c$ or radioactive halogen labeled forms of compounds including various thioflavin derivatives such as 6-iodo-2-[4'-(N,N-dimethylamino)phenyl]benzothiazole (hereinafter referred to as TZDM) and 6-hydroxy-2-[4'-(N-methylamino)phenyl]benzothiazoie (hereinafter referred to as 6-OH-BTA-1) (Patent Document 1, Non-Patent Document 3); stilbene compounds such as (E)-4-methylamino-4'-hydroxystilbene (hereinafter referred to as SB-13) and (E)-4-dimethylamino-4'-iodostilbene (hereinafter referred to as m-I-SB) (Patent Document 2, Mon-Patent Document 4, Non-Patent Document 5); benzoxazole derivatives such as 6-iodo-2-[4'-(N,N-dimethylamino)phenyl]benzoxazole (hereinafter referred to as IBOX) and 6-[2-(fluoro)ethoxy]-2-[2-(2-dimethylaminothiazol-5-yl)ethenyl]benzoxazole(Non-Patent Document 6, Non-Patent Document 7), DDNP derivatives such as 2-(1-{6-[(2-fluoroethyl)(methyl)amino]-2-naphthyl}ethylidene)malononitrile (hereinafter referred to as FDDNP) (Patent Document 4, Non-Patent Document 8); and imidazopyridine derivatives such as 6-iodo-2-[4'-(N,N-dimethylamino)phenyl]imidazo[1,2-a]pyridine (hereinafter referred to as IMPY) (Patent Document 3, Non-Patent Document 9). Further, some of these probes for imaging diagnosis have been studied on human imaging and have been reported to show a significant accumulation in AD patient's brain compared with normal persons (Non-Patent Document 10, Non-Patent Document 11, Non-Patent Document 12, Non-Patent Document 13).

International Publication No. WO2007/002540 pamphlet discloses a series of compounds with a group having affinity with amyloid, to which a radioisotope labeling site is attached via ethylene glycol or polyethylene glycol (Patent Document 5).

International Publication No. WO2007/063946 pamphlet discloses a series of compounds to which a five-membered aromatic heterocyclic group is attached in order to prevent them from being metabolized in brain (Patent Document 6).

[Patent Document 1] JP-T-2004-506723
[Patent Document 2] JP-T-2005-504055
[Patent Document 3] JP-T-2005-512945
[Patent Document 4] JP-T-2002-523383
[Patent Document 5] International Publication No. WO2007/002540 pamphlet
[Patent Document 6] International Publication No. WO2007/063946 pamphlet
[Non-Patent Document 1] J. A. Hardy & G. A. Higgins, "Alzheimer's Disease: The Amyloid Cascade Hypothesis.", Science, 1992, 256, p.184-185
[Non-Patent Document 2] G. McKhann et al., "Clinical diagnosis of Alzheimer's disease: Report of the NINCDS-ADRDA Work Group under the auspices of Department of Health and Human Services Task Force on Alzheimer's Disease.", Neurology, 1984, 34, p.939-944
[Non-Patent Document 3] Z.-P. Zhuang et al., "Radioiodinated Styrylbenzenes and Thioflavins as Probes for Amyloid Aggregates.", J. Med. Chem., 2001, 44, p.1905-1914
[Non-Patent Document 4] Masahiro Ono et al., "11C-labeled stilbene derivatives as Aβ-aggregate-specific PET imaging agents for Alzheimer's disease.", Nuclear Medicine and Biology, 2003, 30, p.565-571
[Non-Patent Document 5] H. F. Kung et al., "Novel Stilbenes as Probes for amyloid plaques.", J. American Chemical Society, 2001, 123, p.12740-12741
[Non-Patent Document 6] Zhi-Ping Zhuang et al., "IBOX(2-(4'-dimethylaminophenyl)-6-iodobensoxazole): a ligand for imaging amyloid plaques in the braid.", Nuclear Medicine and Biology, 2001, 28, p.887-894
[Non-Patent Document 7] Furumoto Y et al., "[11C-]BF-227: A New 11C-Labeled 2-Ethenylbenzoxazole Derivative for Amylcid-β Plaques Tmaging.", European Journal of Nuclear Medicine and Molecular Imaging, 2005, 32, Sup.1, P759
[Non-Patent Document 8] Eric D. Agdeppa et al., "2-Dialkylamino-6-Acylmal.ononitrile Substituted Naphtalenes (DDNP Analogs): Novel Diagnostic and Therapeutic Tools in Alzheimer's Disease.", Molecular Imaging and Biology, 2003, 5, p.404-417
[Non-Patent Document 9] Zhi-Ping Zhuang et al., "Structure-Activity Relationship of Imidazo[1,2-a]pyridines as Ligands for Detecting β-Amyloid Plaques in the Brain.", J. Med. Chem, 2003, 46, p.237-243
[Non-Patent Document 10] W. E. Klunk et al., "Imaging brain amyloid in Alzheimer's disease with Pittsburgh Compound-B.", Ann. Neurol., 2004, 55, p.306-319
[Non-Patent Document 11] Nicolaas P. L. G. Verhoeff et al., "In-Vivo Imaging of Alzheimer Disease β-Amyloid With [11C]SB-13 PET.", American Journal of Geriatric Psychiatry, 2004, 12, p.584-595
[Non-Patent Document 12] Hiroyuki Aral et al., "[11C]-BF-227 AND PET to Visualize Amyloid in Alzheimer's Disease Patients", Alzheimer's & Dementia: The Journal of the Alzheimer's Association, 2006, 2, Sup. 1, S312
[Non-Patent Document 13] Christopher M. Clark et al., "Imaging Amyloid with I123 IMPY SPECT", Alzheimer's & Dementia: The Journal of the Alzheimer's Association, 2006, 2, Sup. 1, S342

## DISCLOSURE OF THE INVENTION

## PROBLEMS TO BE SOLVED BY THE INVENTION

As described above, various compounds are disclosed as probes for imaging diagnosis for amyloid, and researched for clinical application.
Experiments in normal mice show that [$^{125}$I]-labeled TZDM, IBOX and m-I-SB are all transferred into brain 2 minutes after administration. However, these compounds are ins-efficient in clearance from normal tissues, and tend to accumulate gradually in brain as time passes after administration (JP-T-2005-512945; Zhi-Ping Zhuang et al., Nuclear Medicine and Biology, 2001, 28, p.887-894; H. F. Kung et al., J. Am. Chem. Soc., 2001, 123, p.12740-12741). When the clearance from normal tissues is insufficient, a problem arises in that sufficient contrast cannot be obtained at amyloid accumulation sites. [$^{11}$C]-labeled SB-13 shows a clearance from normal tissues in experiments in rats, however, it cannot be said that the clearance is sufficiently fast (Masahiro Ono et al., Nuclear Medicine and Biology, 2003, 30, p.565-571).

Meanwhile, it is revealed that compounds having an imidazopyridine skeleton such as IMPY have a property of transferring to brain and accumulating at amyloid after administration, and also have an excellent property of rapid clearance from normal tissues unlike the above-described compounds, as a result of experiments using [$^{125}$I]-labeled compounds. However, IMPY is a compound positive in reverse mutation test. In order to use this compound as a probe for imaging diagnosis, sufficient care must be taken about dosage and administration manner (International Publication No. WO03/106439 pamphlet).
FDDNP is also reported to be positive in reverse mutation test. (International Publication No. MO03/106439 pamphlet)

The present invention has been made under such circumstances where various compounds have been disclosed as probes targeting amyloid for imaging diagnosis, but there has been no compound which is confirmed to have

a clinically tolerable property, and aims at providing a compound that is effective as a probe targeting amyloid for imaging diagnosis and a reagent for amyloid deposited on a biological tissue the compound.

MEANS FOR SOLVING THE PROBLEMS

As a result of intensive studies, the inventors have found that a compound effective as a probe targeting amyloid for imaging diagnosis can be provided by a series of compound with an imidazopyridine-phenyl skeleton to which a substituent other than hydrogen atom is attached to the carbon atom at 3-position, and thus have completed the present invention.

According to one aspect of the present invention, a compound represented by the following formula (1), and a salt thereof:

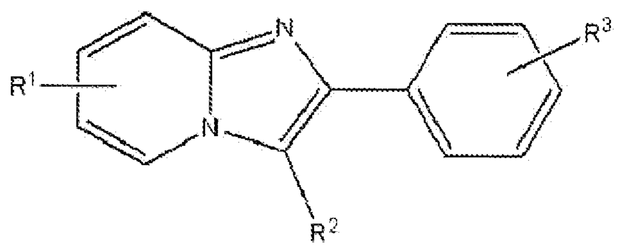

(1)

and a reagent for detecting amyloid deposited on a biological tissue comprising a compound represented by the above formula (1) or a salt thereof are provided. Here, a biological tissue can be various tissues at which amyloid is known to deposit in amyloidosis. Typical examples of such biological tissues include brain, heart, lung, pancreas, bone and joint, and as the most typical biological tissue, mention may be made of brain. The typical amyloidosis in case of brain includes Alzheimer's disease and dementia with Lewy bodies.

In the formula (1), $R^1$ is a group selected from the group consisting of a halogen substituent, an alkyl substituent with 1 to 4 carbon atom, an alkoxy substituent with an alky chain having 1 to 4 carbon atoms and a hydroxyl group, and $R^2$ is a group selected from the group consisting of a cyano substituent, an alkyl substituent with 1 to 4 carbon atoms and a halogen substituent.

$R^3$ is a group selected from the group consisting of a hydroxyl group, a halogen substituent and a substituent represented by the following formula (2):

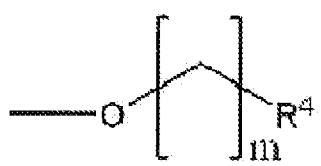

(2)

Meanwhile, in the formula (2), $R^4$ is a hydrogen, a halogen substituent or a hydroxy group, and m is an integer of 1 to 4.

In addition, since the compound of the present invention is used as a probe for image diagnosis, at least one of $R^1$, $R^2$, $R^3$ and $R^4$ is required to be a radioactive halogen. As the radioactive halogen, a nuclide usually used in SPECT and PET can be used. More concretely, a radioactive halogen selected from the group consisting of $^{18}F$, $^{16}Br$, $^{123}I$, $^{124}I$, $^{125}I$ and $^{131}I$ can be used, and more preferably $^{18}F$ or $^{123}I$ can be used.

Therefore, according to the preferable embodiment, a compound of the present invention is selected from the group consisting of 2-(4'-fluorophenyl)-6-[$^{123}$I]iodo-3-methylimidazo[1,2-a]pyridine, 2-[4'-(2"-hydroxyethoxy)phenyl]-6-[$^{123}$I]iodo-3-methylimidazo[1,2-alpyridine, 2-(4'-hydyoxyphenyl)-6-[$^{123}$I]iodo-3-methylimidazo[1,2-a]pyridine, 3-cyano-2-[4'-(2"-hydroxyethoxy)phenyl-"6-[$^{123}$I]iodoimidazo[1,2]pyridine, 2-[4'-(2"-[$^{123}$F]fluoroethoxy)phenyl]-3-iodo-6-methoxyimidazo[1,2-a]pyridine, 2-[4'-(2"-[$^{123}$F]fluoroethoxy)phenyl]-6-methoxy-3-methylimidazo[1,2-a]pyridine and

2-(4'-[$^{18}$F]fluorophenyl)-6-iodo-3-methylimidazo[1,2-a]pyridine, and a detecting reagent of the present invention comprises these compounds or a salt thereof.

According to another aspect of the present invention, a compound represented by any of the following formula (3):

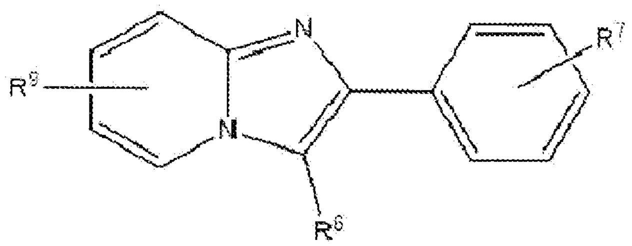

(3)

the following formula (5):

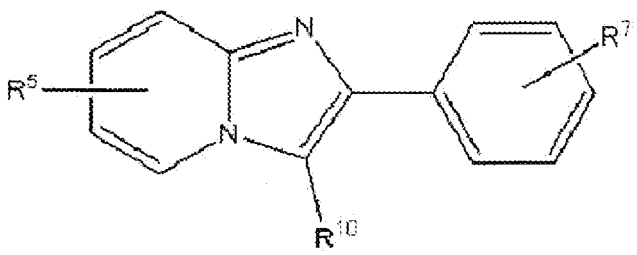

(5)

the following formula (6):

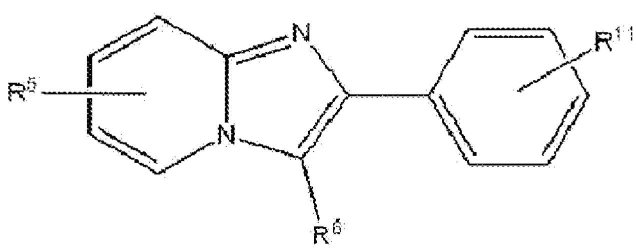

(6)

or the following formula (7):

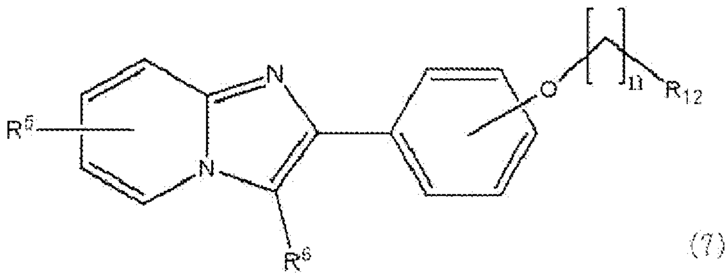

(7)

or a salt thereof is provided.

In the formulas, $R^5$ is a group selected from the group consisting of a a halogen substituent, at alkyl substituent with 1 to 4 carbon atoms, an alkoxy substituent with an alky chain having 1 to 4 carbon, atoms and a hydroxyl group, $R^5$ is a group selected from the group consisting of a cyano substituent, an alkyl substituent with 1 to 4 carbon atoms and a halogen substituent, and $R^7$ is a group selected from the group consisting of a hydroxyl group, a halogen substituent and a substituent represented by the following formula (4) :

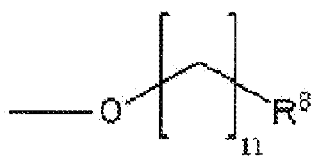

(4)

wherein $R^8$ is a hydrogen, a halogen substituent or a hydroxyl group, and n is an integer of 1 to 4.

$R^9$ is a group selected from the group consisting of a non-radioactive halogen substituent, a nitro group, a trialkylstannyl substituent having alkyl chains with 1 to 4 carbon atoms or a group, $R^{10}$ is a group selected from the group consisting of a non-radioactive halogen substituent, a nitro group, a trialkylstannyl having alkyl chains with 1 to 4 carbon atoms or a triphenylstannyl group, $R^{11}$ is a group selected from the group consisting of a non-radioactive halogen substituent, a nitro substituent, a trialkylstannyl substituent having alkyl chains with 1 to 4 carbon atoms or a triphenylstannyl group, and $R^{12}$ is a group selected from the group consisting of at non-radioactive halogen substituent, a methanesulfonyloxy substituent, a trifluoromethanesulfonyloxy substituent and an aromatic sulfonyloxy substituent.

## EFFECTS OF THE INVENTION

The present invention provides a novel compound having affinity with amyloid any having an excellent capability of imaging amyloid in a living body, and a reagent for detecting amyloid deposited on a biological tissue.

## BEST MODE FOR CARRYING OUT THE INVENTION

(Synthesis of a precursor compound for a radioactive halogen-labeled compound)

Hereinafter, a method for synthesis of a compound according to the present invention will be described, taking the case of 6-tributylstannyl-2-[4'-(2"-hydroxyethoxy)phenyl]-3-methylimidazo[1,2-a]pyridine as an example.

For the synthesis of 6-tributylstannyl-2-[4'-(2"-hydroxyethoxy)phenyl]-3-methylimidazo[1,2-a]pyridine, firsts 4'-hydroxypropioyphenone is allowed to react with cupric bromide to prepare 2-bromo-4'-bydroxypropiophenone (Fig. 1, Step 1). This reaction can be conducted in accordance with ordinary methods, for example, the method described in a literature, King, L. Carroll & Ostrum, G. Kenneth, Journal of Organic Chemistry, 1964, 29(12), p.3459-8461.

Then, 2-'bromo-4'-hydroxypropiophenone as prepared above is allowed to react with 2-amino-5-lodopyridine to prepare 2-(4'-hydrXxyphenl)-6-iodo-3-methylimidazo [1,2-alpyridine (Fig. 1, Step 2). This step can be done, for example, according to the following procedure.

First, 2-bromo-4'-hydroxypropiophencne and 2-amino-5-iodopyridine are dissolved in an inactive solvent such as acetonitrile, and are allowed to react with each other at a reflux temperature four 2 to 6 hours to produce 2-(4'-hydroxyphenyl-6-iodo-3-methylimidazo-[1,2-a]pyridine hydrobromide salt as white precipitates. The solvent used in this

instance may be acetonitrile or another solvent that is usually employed in a similar reaction, for example, methanol and acetone. The reaction temperature may be a temperature allowing refluxing, for example, 110˚C when the solvent is acetonitrile. The amount of the solvent to be used may be an amount sufficient to effect the reaction, however, it should be noted that if the solvent is too much, it will become difficult to obtain precipitates of reaction products. For example, when 2-bromo-4'-hydroxypropicphenone in an amount corresponding to 10 mmol is used for the reaction, the amount of a solvent to be used can be about 40 to 80 mL.

Next, the reaction solution is filtered to recover the precipitates. The white precipitates are suspended in a mixed solution of methanol/water (1:1). Then, an aqueous saturates solution of sodium hydrogencarbonate is added thereto in a very excessive amount relative to the suspended precipitates to release 2-(4'-hydroxyphenyl)-6-iodo-3-methylimidazo[1,2-a]pyridine as precipitates. The newly generated precipitates are filtered to recover 2-(4'-"hydroxyphenyl)-6-iodo-3-methylimidazo[1,2-a]pyridine as the target compound in this step (Fig. 1, Step 2). The amount of the mixed solution of methanol/water is not specifically limited as long as it is sufficient to effect the reaction. However, it should be noted that if the amount of the mixed solution is too much, precipitation of products will be hindered. For example, when 2-bromo-4' -hydroxypropiophenone in an amount corresponding to 10 mmol is used, the mixed solution of methanol/water may be used in an amount of about 40 to 100 mL. The amount of sodium hydrogencarbonate is not specifically limited as long as it is very excessive relative to the above-described precipitates as reaction substrates. For example, when the reaction is effected under the above-described conditions, the amount of an aqueous saturated solution of sodium hydrogencarbonate to be added to the reaction solution can be about 50 mL.

Here, 2-bromoethanol and t-butyldiphenylchlorosilane (TBDPSC1) are reacted with each other to prepare 1-bromo-2-(t-butyldiphenylsiloxy)ethane (Fig. 1, Step 3), separately. In this instance, the reaction can be carried out in accordance with ordinary methods, for example, the method described in a literature (Organic Syntheses, Coll. Vol. 10, p.170 (2004); Vol. 79, p.59 (2002)).

Then, the 2-(4'-hydroxyphenyl)-6-iodo-3-methylimidazo[1,2-a]pyridine prepared above is sufficiently dried, dissolved in N,N-dimethylformamide, and potassium carbonate and 1-bro-mo-2-(t-butyldiphenylsiloxy) ethane were added thereto. After this mixture was stirred at about 90˚C for about 2 hours, a saturated sodium chloride solution is added followed by extraction with ethyl acetate, and the ethyl acetate layer is concentrated and subjected to chromatogram purification to obtain 2-[4'-(2"-t-butyldiphenylsiloxyethoxy)phenyl]-6-iodo-3-methylimidazo[1,2-a]pyridine (Fig. 1, Step 4). The amount of potassium carbonate may be an amount that can neutralize hydrobromic acid generated from 1-bromo-2-(t-butyldiphenylsiloxy) ethane during the reaction, and is typically about double to triple the other reactant 1-bromo-2-(t-butyldiphenylsiloxy)ethane in molar ratio. Further, the 1-bromo-2-(t-butyldiphenylsiloxy)ethane can be used in an excessive amount relative to the reaction substrate, and is typically about 1.5 times the reaction substrate 2-(4'-hydroxyphenyl)-6-iodo-3-methylimidazo[1,2-a]pyridine in molar ratio.

Then, t-butyldiphenylsilyl group of the obtained 2-[4'-(2"-t-butyldiphenylsiloxyethoxy)phenyl]-6-iodo-3-methylimidazo[1,2-a]pyridine is deprotected using tetrabutylammonium fluoride to obtain 2-[4'-(2"-hydroxyethoxy)phenyl]-6-iodo-3-methylimidazo[1,2-a]pyridine (Fig. 1, Step 5). In this instance, the reaction can be carried out in accordance with ordinary methods, for example, the method described in a literature (Organic Syntheses, Coll. Vol. 9, p.417 (1998); Vol. 74, p.248 (1997)).

The obtained 2-[4'-(2"-hydroxyethoxy)]phenyl]-6-iodo-3-methylimidazo[1,2-a]pyridine is dissolved in dioxane, and triethylamine is added to the solution, followed by addition of bis(tributyltin) and a catalytic amount of tetrakis-triphenylphosphine palladium. This reaction solution is heated at about 90˚C to effect reaction for about 24 hours, and then a solvent is distilled off and chromatographic purification is performed to obtain 6-tributylstannyl-2-[4'-(2"-hydroxyethoxy)]phenyl]-3-methylimidazo[1,2-a]pyridine as the target compound (Fig. 2, Step 1). The amount of bis(tributyltin) to be used in this instance may be an amount satisfying a condition where it is excessive relative to the reaction substrate, specifically, it is about 1.5 times in molar ratio relative to the reaction substrate 2-[4'-(2"-hydroxyethoxy)phenyl]-6-iodo-3-methylimidazo[1,2-a]pyridine.

When a compound with a substituent at the 6-position in the imidazo pyridine ring being a trialkylstannyl substituent other than the tributylstannyl substituent is obtained, various bis(trialkyltin)s that fit purposes can be used instead of bis(tributyltin) in Fig. 2, Step 1. For example, when a compound having a trimethylstannyl substituent as a substituent at the 6-position is synthesized, a reaction similar to the above can be performed using bis(trimethyltin) in Fig. 2, Step 1.

A compound with an imidazopyridine ring in which the binding site for the functional group is a carbon atom other than the carbon at 6-position can be obtained by using a compound with a pyridine ring to which iodine is bonded at a different site instead of 2-amino-5-iodopyridine in Fig. 1, Step 2. For example, when a binding site for the functional group is the carbon at 3-position in the imidazopyridine ring, 2-amino-3-iodopyridine may be used instead of 2-amino-5-iodopyridine in Fig. 1, Step 2.

(A method for synthesis of a radioactive halogen-labeled compound)

Next, a method for production of a radioactive halogen-labeled compound according to another aspect of the present invention will be described, taking the case of radioactive iodine-labeled compounds as an example.

The synthesis of radioactive iodine-labeled compounds can be performed by dissolving the labeling precursor compound prepared as above procedure in an inert organic solvent, adding thereto a [$^{123}$I] sodium iodide solution or the like obtained by known methods, and adding thereto an acid and an oxidizing agent to effect the reaction. As an inert organic solvent dissolving the labeling precursor compound, various solvents having no reactivity with the labeling precursor, [$^{123}$I]sodium iodide and the like can be used, and preferably methanol can be used.

As the acid, may be used various ones, and preferably hydrochloric acid.
The oxidizing agent is not particularly limited as long as it can effect the oxidation of iodine in the reaction solution, and is preferably hydrogen peroxide or peracetic acid. The amount of the oxidizing agent to be added may be an amount sufficient to oxidize iodine in the reaction solution.

A compound labeled with a radioactive halogen other than iodine can be synthesized by labeling a labeling precursor that fits a purpose of synthesis with a radioactive halogen that fits the purpose. For example, in order to synthesise 2-(4'-[$^{18}$F]fluorophenyl)-6-iodo-3-methylimidazo[1,2-a]pyridine, the labeling precursor 2-(4' -nitrophenyl)-6-iodo-3-methylimidazo[1,2-a]pyridine can be reacted with [$^{18}$F]fluoride ion in the presence of a phase transfer catalyst and potassium carbonate.

(Methods for preparing and using a diagnostic agent in accordance with the present invention)

The diagnostic agent according to the present invention can be prepared as a solution which comprises the present radioactive halogen-labeled compound blended in water, a physiological saline solution or a Ringer's solution optionally adjusted to an appropriate pH, like other commonly-known radioactive diagnostic agent. In this instance, concentration of the present compound should be adjusted to not more than the concentration at which stability of the present compound is ensured. Dosage of the present compound is not specifically limited as long as it is sufficient to obtain an image of distribution of an administered agent. For example, in case of iodine-123 ($^{123}$I)-labeled compounds and fluorine-18 ($^{18}$F)-labeled compounds, about 50 to 600 MBq per adult body of 60 kg weight can be administered intravenously or locally. Distribution of administered agents can be imaged by known methods. For example, iodine-123($^{123}$I)-labeled compounds can be imaged by a SPECT apparatus while fluorine-18($^{18}$F)-labeled compounds can be imaged by a PET apparatus.

Hereinafter, the present invention is described below in more detail by way of Examples, Comparative Examples and Reference Examples, my However, these Examples never limit the scope of the present invention. Meanwhile, in the following Examples, the names of the individual compounds used in the experiment are defined as shown in Table 1.

Table 1

| Compound name | Common name |
|---|---|
| Compound 1 | 2-(4'-fluorophenyl)-6-[$^{123}$I]iodo-3-methylimidazo[1,2-a]pyridine |
| Compound 2 | 2-[4'-(2"-hydroxyethoxy)phenyl]-6-[$^{123}$I]iodo-3-methylimidazo[1,2-a]pyridine |
| Compound 3 | 2-(4'-hydroxyphenyl)-6-[$^{123}$I]iodo-3-methylimidazo[1,2-a]pyridine |
| Compound 4 | 3-cyano-2-[4'-(2"-hydroxyethoxy)phenyl]-6-[$^{123}$I]iodoimidazo[1,2-a]pyridine |
| Compound 5 | 2-[4'-(2"-hydroxyethoxy)phenyl]-6-iodo-3- methylimidazo[1,2-a]pyridine (non-radioactive iodine- labeled form) |

<u>Example 1: Synthesis of 2-[4'-(2"-hydroxyethoxy)phenyl]-6-iodo-3-methylimidazo[1,2-a]pyridine (non-radioactive iodinated form)</u>

40 mL of ethyl acetate was added to 6.26 g (corresponding to 27.9 mmol) of cupric bromide to obtain a suspension, to which 2.00 g (corresponding to 13.3 mmol) of 4'-hydroxypropiophenone was added. Then, the resulting mixture was heated under reflux. After 2 hours, the reaction mixture was cooled down to room temperature and filtered. The resulting filtrate was concentrated under reduced pressure. The resulting crude product was purified by flash silica gel column chromatography (elution solvent: a hexane/ethyl acetate = 3/1), to obtain 3.49 g (corresponding to 15.2 mmol) of 2-bromo-4'-, hydroxypropiophenone (Fig. 1, Step 1).

734 mg (corresponding to 3.20 mmol) of 2-bromo-4'-hydroxypropiophenone and 705 mg (corresponding to 3.20 mmol) of 2-amino-5-iodopyridine were dissolved in 15 mL of acetonitrile. The resulting solution was heated under relux in an oil bath at 110°C for 4 hours. After the completion of the reaction, the reaction solution was cooled down to room temperature, and precipitates were filtered and recovered. The precipitates were washed with acetonitrile and dried under reduced pressure. The resulting crude crystals were suspended in a mixed solution of 3.0 mL of water and 1.0 mL of methanol. Then, about 10 mL of a saturated sodium hydrogencarbonate solution was added thereto, and the mixture was sonicated for 5 minutes using an ultrasonic washing machine. Precipitates were filtered and recovered from the resulting mixture, sufficiently washed with water, and dried under reduced pressure, to obtain 226 mg (corresponding to 0.645 mmol) of 2-(4'-hydroxyphenyl)-6-iodo-3-methylimidazo[1,2-a]pyridine (Fig. 1, Step 2).

Separately, 2.50 g (corresponding to 20.0 mmol) of 2-bromoethanol and 2.72 g (corresponding to 40.0 mmol) of imidazole were dissolved in 10 mL of N,N-dimethylformamide (DMF), and cooled to 0°C. Then, 5.50 g (corresponding to 20.0 mmol) of t-butyldipbenylchlorosilane (TBDPSC1) was added thereto. After the reaction mixture was stirred at room temperature for 18 hours, a saturated sodium chloride aqueous solution was added, and extracted three times with ethyl acetate. The combined ethyl acetate layers were dried over anhydrous sodium sulfate, and concentrated under reduced pressure. The resulting crude product was purified by silica gel column chromatography (elution solvent: hexane/ethyl acetate = 10/1) to obtain 7.04 g (corresponding to 19.4 mmol) of 1-bromo-2-(t-butyldiphenylsiloxy)ethane (Fig. 1, Step 3).

220 mg (corresponding to 0.628 mmol) of 2-(4'-hydroxyphenyl)-6-iodo-3-methylimidazo[1,2-a]pyridine was dissolved in 3.0 mL of N,N-dimethylformamide, and 260 mg (corresponding to 1.88 mmol) of potassium carbonate was added thereto. Then, 251 mg (corresponding to 0.691 mmol) of 1-bromo-2-(t-butyldiphenylsiloxy)ethane was added thereto. After the reaction mixture was stirred at 90°C for 2 hours, a saturated ammonium chloride aqueous solution was added, and extracted three times with ethyl acetate. The combined ethyl acetate layers were dried over anhydrous sodium sulfate, and concentrated under reduced pressure. The resulting crude product was purified by silica gel column chromatography (elution solvent: hexane/ethyl acetate = 3/1) to obtain 344 mg (corresponding to 0.544 mmol) of 2-[4'-(2"-t-butyldiphenylsiloxyethoxy)phenyl]-6-iodo-3-methylimidazo[1,2-a]pyridine (Fig. 1, Step 4).

344 mg (corresponding to 0.544 mmol) of 2-[4'-(2"-t-butyldiphenylsiloxyethoxy)phenyl]-6-iodo-3-methylimidazo [1,2-a]pyridine was dissolved in 1.0 mL of tetrahydrofuran, and 0.65 mL of a 1.0 mol/L solution in tetrahydrofuran of tetrabutylammoniumfluoride was added thereto. After the reaction mixture was stirred at room temperature for 30 minutes, ammonium chloride aqueous solution was added, followed by addition of 5.0 mL of water and 2.0 mL of acetonitrile. Then precipitates were filtered. The filtered precipitates were washed with water and acetonitrile in this order, to obtain 166 mg (corresponding to 0.421 mmol) of 2-[4'-(2"-hydroxyethoxy)phenyl]-6-iodo-3-methylimidazo[1,2-a]pyridine (Fig. 1, Step 5).

The NMR measurement results of the resulting 2-[4'-(2"-hydroxyethoxy)phenyl]-6-iodo-3-methylimidazo[1,2-a]pyridine (internal standard: tetramethylsilane) are shown below.

NMR apparatus employed: JNM-ECP-500 (manufactured by Japan Electron Optics Laboratory Co., Ltd. (JEOL))

$^1$H-NMR (solvent: dimethylsulfoxide-d6; resonance frequency: 500 MHz) : δ 8.59 (s, 1H), 7.01 (d, J = 7.3 Hz, 2H), 7.38 (s, 2H), 7.03 (d, J = 7.3 Hz, 2H), 4.87 (t, J = 5.5 Hz, 1H), 4.02 (t, J = 5.0 Hz, 2H), 3.73 (dt, J = 5.5, 5.0 Hz, 2H), 2.60 (s, 2H).

$^{13}$C-NMR (solvent: dimethylsulfoxide-d6, resonance frequency: 125 MHz): δ 158.9, 142.7, 142.1, 131.8, 129.8, 129.6, 127.6, 118.4, 116.6, 115.3, 76.3, 70.3, 60.4, 10.2.

Example 2: Synthesis of 6-tributylstannyl-2-[4'-(2"-hydroxyethoxy)phenyl]-3-methylimidazo[1,2-a]pyridine

50.0 mg (corresponding to 0.127 mmol) of 2-[4'-(2"-hydroxyethoxy)phenyl]-6-iodo-3-methylimidazo[1,2-a]pyridine obtained in Example 1 was dissolved in 3.0 mL of dioxane, and 0.50 mL of triethylamine was added thereto. Then, 0.10 mL (corresponding to 0.19 mmol) of bis(tributyltin) and 9.7 mg (a catalytic amount) of tetrakis-triphenylphosphine palladium were added thereto. After the reaction mixture was stirred at 90°C for 18 hours, the solvent was distilled off under reduced pressure. The residue was purified by flash silica gel column chromatography (elution solvent: hexane/ethyl acetate = 2/1), to obtain 52.9 mg (corresponding to 0.0949 mmol) of 6-tributylstannyl-2-[4'-(2"-hydroxyethoxy)phenyl]-3-methylimidazo[1,2-a]pyridine (Fig. 2, Step 1).

The NMR measurement results of the resulting 6-tributylstannyl-2-[4'-(2"-hydroxyethoxy)phenyl]-3-methylimidazo[1,2-a]pyridine (internal standard: tetramethylsilane) are shown below.

NMR apparatus employed: JMM-ECP-500 (manufactured by Japan Electron Optics Laboratory Co., Ltd. (JEOL))

$^1$H-NMR (solvent: chloroform-d1; resonance frequency: 500 MHz): δ 7.76-7.65 (m, 3H), 7.61 (d, J = 8.7 Hz, 1H), 7.18 (d, J = 8.7 Hz, 1H), 7.00 (d, J = 10.2 Hz, 2H), 4.10 (t, J = 4.6 Hz, 2H), 3.98 (t, J = 4.6 Hz, 2H), 2.63 (s, 3H), 1.64-1.51 (m, 6H), 1.40-1.32 (m, 6H), 1.20-1.10 (m, 6H), 0.91 (t, J = 7.4 Hz, 9H).

Example 3: Synthesis of 2-[4'-(2''-hydroxyethoxy)phenyl]-6-[$^{123}$I]iodo-3-methylimidazo[1,2-a]pyridine

To 60 μL of a solution of 6-tributylstannyl-2-[4'-(2''-hydroxyethoxy)phenyl]-3-methylimidazo[1,2-a]pyridine (concentration: 1 mg/mL) in a mixed solution of methanol/dimethylsulfoxide with mixing ratio of 9/1, 30 μL of 1 mol/L hydrochloric acid, 15 μL of 1 mmol/L sodium iodide, 18 μL of [$^{123}$I] sodium iodide of 303 MBq and 15 μL of 10 % (w/v) hydrogen peroxide were added. After the mixed solution was left to stand at 50°C for 10 minutes, it was subjected to HPLC under the following conditions, to obtain a fraction of 2-[4'-(2''-hydroxyethoxy)phenyl]-6-[$^{123}$I]iodo-3-methylimidazo[1,2-a]pyridine.

HPLC conditions:

Column: Phenomenex Luna C18 (trade name; manufactured by Phenomenex Co.; size: 4.6 x 150 mm)
Mobile phase: 0.1 % trifluoroacetic acid in water/0.1 % trifluoroacetic acid in acetonitrile = 20/80 to 0/100 (17 minutes)
Flow rate: 1.0 mL/min.
Detector: Ultraviolet visible absorptiometer (Detection wavelength: 282 nm) and radioactivity counter (manufactured by raytest: type STEFFI)

10 ml of water was added to the fraction. The resulting solution was passed through a reversed phase column (trade name: Sep-Pak (registered trademark) Light C8 Cartridges manufactured by Waters: the packed amount of the packing agent: 145 mg) so that the column adsorbs and collects 2-[4'-(2''-hydroxyethoxy)phenyl]-6-[$^{123}$I]iodo-3-methylimidazo[1,2-a]pyridine. The column was rinsed with 1 mL of water, and then 1 mL of diethyl ether was passed therethrough to elute 2-[4'-(2''-hydroxyethoxy)phenyl]-6-[$^{123}$I]iodo-3-methylimidazo[1,2-a]pyridine. The amount of radioactivity of the obtained compound was 184.5 MBq at the end of synthesis. Further, the TLC analysis was conducted under the following conditions, and as a result, the radiochemical purity of the compound was 99%.

TLC analysis conditions:

TLC plate: Silica Gel 60 $F_{254}$ (trade name; manufactured by Merck & Co., Inc.)
Mobile phase: Chloroform/methanol/triethylamine = 100/1/2 Detector: Rita Star (trade name; manufactured by raytest)

Example 4: Synthesis of 2-(4'-fluorophenyl)-6-iodo-3-methylimidazo[1,2-a]pyridine (non-radioactive iodinated form)

20 mL of ethyl acetate was added to 1.50 g (corresponding to 6.44 mmol) of cupric bromide to obtain a suspension, to which 426 mg (corresponding to 2.80 mmol) of 4'-fluoropropiophenone was added. Then, the mixture was heated under reflux. After 2 hours, the reaction solution was cooled down to room temperature and filtered. Then, the resulting filtrate was concentrated under reduced pressure. The residue dissolved in ethyl acetate, and the resulting crude product was purified by flash silica gel column chromatography (elution solvent: hexane/ethyl acetate = 10/1), to obtain 650 mg (corresponding to 2.80 mmol) of 2-bromo-4'-fluoropropiophenone (Fig. 3, Step 1).

650 mg (corresponding to 2.80 mmol) of 2-bromo-4'-fluoropropiophenone and 616 mg (corresponding to 2.30 mmol) of 2-amino-5-iodopyridine were dissolved in 15 mL of acetonitrile. The resulting solution was heated under reflux in an oil bath at 110°C for 2 hours. After the completion of the reaction, the reaction solution was cooled down to room temperature, and precipitates were filtered. Then, the precipitates were washed with acetonitrile and dried under reduced pressure. The resulting crude crystals were suspended in a mixed solution of 1.0 mL of water and 1.0 mL of methanol. Then, about 1.0 mL of a saturated sodium hydrogencarbonate solution was added thereto, and the mixture was sonicated for 10 minutes using an ultrasonic washing machine. Precipitates were filtered and recovered from the resulting mixture, sufficiently washed with water, and dried under reduced pressure, to obtain 42.8 mg (corresponding to 0.122 mmol) of 2-(4'-fluorophenyl)-6-iodo-3-methylimidazo[1,2-a]pyridine (Fig. 3, Step 2).

The NMR measurement results of the resulting 2-(4'-fluorophenyl)-6-iodo-3-methylimidazo[1,2-a]pyridine (internal standard: tetramehtylsilane) are shown below.

NMR apparatus employed: JNM-ECP-500 (manufactured by Japan Electron Optics Laboratory Co., Ltd. (JEOL))
$^1$H-NMR (solvent: chloroform-d1; resonance frequency: 500 MHz): δ 8.17 (s, 1H), 7.76-7.74 (m, 2H), 7.46 (d, J = 9.2 Hz, 1H), 7.37 (d, J = 9.2 Hz, 1H), 7.19-7.15 (m, 2H), 2.62 (s, 3H).

Example 5: Synthesis of 6-tributylstannyl-2-(4'-fluorophenyl)-2-methylimidazo[1,2-a]pyridine

22.5 mg (corresponding to 63.9 μmol) of 2-(4'-fluorophenyl)-6-iodo-3-methylimidazo[1,2-a]pyridine obtained in Example 4 was dissolved in 2.0 mL of dioxane, and 1.0mL of triethylamine was added thereto. Then, 0.048 mL (corresponding to 96 μmol) of bis(tributyltin) and 0.6 mg (a catalytic amount) of tetrakis-triphenylphosphine palladium were added thereto. After the reaction mixture was stirred at 90°C for 22 hours, the solvent was distilled off under reduced

pressure. The residue was purified by flash silica gel column chromatography (elution solvent: hexane/ethyl acetate = 5/1), to obtain 31.9 mg (corresponding to 61.9 mol) of 6-tributylstannyl-2-(4'-fluorophenyl)-3-methylimidazo[1,2-a]pyridine (Fig. 4, Step 1).

The NMR measurement results of the resulting 6-tributylstannyl-2-(4'-fluorophenyl)-3-methylimidazo[1,2-a] pyridine (internal standard: tetramehtylsilane) are shown below.

NMR apparatus employed: JNM-ECF-500 (manufactured by Japan Electron Optics Laboratory Co., Ltd. (JEOL)) $^1$H-NMR (solvent: chloroform-d1; resonance frequency: 500 MHs): $\delta$ 7.78 (m, 3H), 7.63 (d, J = 8.8 Hz, 1H), 7.19-7.14 (m, 3H), 2.63 (s, 3H), 1.69-1.49 (m, 6H), 1.40-1.34 (m, 6H), 1.20-1.07 (m, 6H), 0.94-0.90 (m, 9H)

$^{13}$C-NMR (solvent: chloroform-d1, resonance frequency: 125 MHz): $\delta$ 144.3, 140.7, 130.9, 130.6, 130:1, 130.0, 127.3, 121.9, 116.8, 115.5, 114.7, 29.0, 27.3, 16.4, 13.7, 9.9.

Example 6: Synthesis of 2-(4'-fluorophenyl)-6-[$^{123}$I]iodo-3-methylimidazo[1,2-a]pyridine

To 60 $\mu$L of a solution of 6-tributylstannyl-2-(4'-fluorophenyl)-3-methylimidazo[1,2-a]pyridine (concentration: 1 mg/mL) in a mixed solution of methanol/dimethylsulfoxide (mixing ratio: 9/1), 70 $\mu$L of 2 mol/L, hydrochloric acid, 15 $\mu$L of 1 mmol/L sodium
iodide, 100 $\mu$L of [$^{123}$I]sodium iodide of 348 MBq and 15 $\mu$L of 10 % (w/v) hydrogen peroxide were added. After the mixed solution was left to stand at 50°C for 10 minutes, it was subjected to HPLC under the following conditions to obtain a fraction of 2-(4'-fluorophenyl)-6-[$^{123}$I]iodo-3-methylimidazo[1,2-a]pyridine.

HPLC conditions:

Column: Phenomenex Luna C18 (trade name; manufactured by Phenomenex Co.; size: 4.6 x 150 mm)
Mobile phase: 0.1 % trifluoroacetic acid in water/0.1 % trifluoroacetic acid in acetonitrile = 20/80 to 0/100 (17 minutes)
Flow rate: 1.0 mL/min.
Detector: Ultraviolet visible absorptiometer (Detection wavelength: 282 nm) and radioactivity counter (manufactured by raytest: type STEFFI)

10 ml of water was added to the fraction. The resulting solution was passed through a reversed phase column (trade name: Sep-Fak (registered trademark) Light C8 Cartridges manufactured by Waters: the packed amount of the packing agent: 145 mg) so that the column adsorbs and collects 2-(4'-fluorophenyl)-6-[$^{123}$I]iodo-3-methylimidazo[1,2-a]pyridine. The column was rinsed with 1 mL of water, and then 1 mL of diethyl ether was passed therethrough to elute 2-(4'-fluorophenyl)-6-[$^{123}$I]iodo-3 methylimidazo[1,2-a]pyridine. The amount of radioactivity of the obtained compound was 114.2 MBq at the end of synthesis. Further, the TLC analysis was conducted under the following conditions, and as a result, the radiochemical purity of the compound was 99%.

TLC analysis conditions:

TLC plate: Silica Gel 60 $F_{254}$ (trade name; manufactured by Merck & Co., Inc.)
Mobile phase: Chloroform/methanol/triethylamine = 100/1/2 Detector: Rita Star (trade name; manufactured by raytest)

Example 7: Synthesis of 2-(4'-hydroxyphenyl)-6-iodo-3-methylimidazo[1,2-a]pyridine (non-radioactive iodinated form)

40 mL of ethyl acetate was added to 6.26 g (corresponding to 27.9 mmol) of cupric bromide to obtain a suspension, to which 2.00 g (corresponding to 13.3 mmol) of 4'-hydroxypropiophenone was added. Then, the mixture was heated under reflux. After 2 hours, the reaction solution was cooled down to room temperature and filtered. Then, the resulting filtrate was concentrated under reduced pressure. The resulting crude product was purified by flash silica gel column chromatography (elution solvent: hexane/ethyl acetate = 3/1), to obtain 3.49 g (corresponding to 15.2 mmol) of 2-bromo-4'-hydroxypropiophenone (Fig. 5, Step 1).

734 mg (corresponding to 3.20 mmol) of 2-bromo-4'-hydroxypropiophenone and 705 mg (corresponding to 3.20 mmol) of 2-amino-5-iodopyridine were dissolved in 15 mL of acetonitrile. The resulting solution was heated under reflux in an oil bath at 110°C for 4 hours. After the completion of the reaction, the reaction solution was cooled down to room temperature, and precipitates were filtered. Then, the precipitates were washed with acetonitrile and dried under reduced pressure. The resulting crude crystals were suspended in a mixed solution of 3.0 mL of water and 1.0 mL of methanol. Then, about 10 mL of a saturated sodium hydrogencarbonate solution was added thereto, and the mixture was sonicated for 5 minutes using an ultrasonic washing machine. Precipitates were filtered and recovered from the resulting mixture, sufficiently washed with water, and dried under reduced pressure, to obtain 226 mg (corresponding to 0.645 mmol) of 2-(4'-hydroxyphenyl)-6-iodo-3-methylimidazo[1,2-a]pyridine (Fig. 5, step 2).

The NMR measurement results of the resulting 2-(4'-hydroxyphenyl)-6-iodo-3-methylimidazo[1,2-a]pyridine (internal standard: tetramehtylsilane) are shown below.

NMR apparatus employed: JNM-ECP-500 (manufactured by Japan Electron Optics Laboratory Co., Ltd. (JEOL)) $^1$H-NMR (solvent: dimethylsulfoxide-d6; resonance frequency: 500 MHz): δ 9,77 (s, 1H), 8.79 (s, 1H), 7.66-7.64 (m, 1H), 7.60-7.58 (m, 2H), 7.51-7.49 (m, 1H), 6.92-6.90 (m, 2H), 2.61 (s, 3H).

Example 8: Synthesis of 6-tributylstannyl-2-(4'-hydroxyphenyl)-3-methylimidazo[1,2-a]pyridine

100 mg (corresponding to 0.286 mmol) of 2-(4'-hydroxyphenyl)-6-icdo-3-methylimidazo[1,2-a]pyridine obtained in Example 7 was dissolved in 4.0 mL of dioxane, and 2.0 mL of triethylamine was added thereto. Then, 0.22 mL (corresponding to 0.43 mmol) of bis(tributyltin) and 1.9.8 mg (a catalytic amount) of tetrakis-triphenylphosphine palladium were added thereto. After the reaction mixture was stirred at 90°C for 78 hours, the solvent was distilled off under reduced pressure. The residue was purified by flash silica gel column chromatography (elution solvent: hexane/ethyl acetate = 1/1), to obtain 55.0 mg (corresponding to 0.107 mmol) of 6-tributylstannyl-2-(4'-hydroxyphenyl)-3-methylimidazo[1,2-a] pyridine (Fig. 6, Step 1).

The NMR measurement results of the resulting 6-tributylstannyl-2-(4'-hydroxyphenyl)-3-methylimidazo[1,2-a] pyridine (internal standard: tetramethylsilane) are shown below.

NMR apparatus employed: JNM-ECP-500 (manufactured by Japan Electron Optics Laboratory Co., Ltd. (JEOL)) $^1$H-NMR (solvent: chloroform-d1; resonance frequency: 500 MHz): δ 7.72 (s, 1H), 7.63 (d, J = 8.7 Hz, 1H), 7.52 (d, J = 8.7 Hz, 2H), 7.17 (d, J = 8.7 Hz, 1H), 6.85 (d, J = 8.7 Hz, 2H), 2.60 (s, 3H), 1.65-1.48 (m, 6H), 1.39-1.32 (m, 6H), 1.19-1.06 (m, 6H), 0.90 (t, J = 7.3 Hz, 9H).

Example 9: Synthesis of 2-(4'-hydroxyphenyl)-6-[$^{123}$I]iodo-3-methylinridazo[1,2-a]pyridine

To 60 μL of a solution of 6-tributylstannyl-2-(4'-flydroxyphenyl)-3-methylimidazo[1,2-a]pyridine (concentration: 1 mg/mL) in a mixed solution of methanol/dimethylsulfoxide (mixing ratio: 9/1), 30 μL of 1 mol/L hydrochloric acid, 15 μL of 1 mmol/L sodium iodide, 18 μL of [$^{123}$I] sodium iodide of 303 MBq and 15 μL of 10 8 (w/v) hydrogen peroxide were added. After the mixed solution was left to stand at 50°C for 10 minutes, it was subjected to HPLC under the following conditions to obtain a fraction of 2-(4'-hydraxyphenyl)- 6-[$^{123}$I]iodo-3-methylimidazo[1,2-a]pyridine.

HPLC conditions:

Column: Phenomenex Luna C18 (trade name; manufactured by Phenomenex Co.; size: 4.6 x 150 mm)
Mobile phase: 0.1 % trifluoroacetic acid in water/0.1 % trifluoroacetic acid in acetonitrile - 20/80 to 0/100 (17 minutes)
Flow rate: 1.0 mL/min.
Detector: Ultraviolet visible absorptiometer (Detection wavelength: 282 nm) and radioactivity counter (manufactured by raptest: type STEFFI)

10 ml of water was added to the fraction. The resulting solution was passed through a reversed phase column (trade name: Sep-Pak (registered trademark) Light C8 Cartridges manufactured by Waters: the packed amount of the packing agent: 145 mg) so that the column adsorbs and collects 2-(4'-hydroxyphenyl)-6-[$^{123}$I]iodo-3-inethylimidazo[1,2-a]pyridine. The column was rinsed with 1 mL of Mater, and then 1 mL of diethyl ether was passed therethrough to elute 2-(4'-hydroxyphenyl)-6-[$^{123}$I]iodo-3-methylimidazo[1,2-a]pyridine. The amount of radioactivity of the obtained compound was 95.7 MBq at the end of syntheses. Further, the TLC analysis was conducted under the following conditions, and as a result, the radiochemical purity of the was 99%.

TLC analysis conditions:

TLC plate: Silica Gel 60 $F_{234}$ (trade name; manufactured by Merck & Co., Inc.)
Mobile phase: Chloroform/methanol/triethylamine = 100/1/2 Detector: Rita Star (trade name; manufactured by raytest)

Example 10: Synthesis of 3-cyano-2-[4'-(2''-hydroxyethoxy)phenyl]-6-iodoimdazo[1,2-a]pyridine (non radioactive iodinated form)

300 mag (corresponding to 2.20 mol) of 4'-hydroxyacetophenone was dissolved in 10.0 mL of dimethylformamide, and 365 mg (corresponding to 2.64 mmol) of potassium carbonate was added thereto. Then, 800 mg (corresponding to 2.20 mmol) of 1-bromo-2-(t-butyldiphenylsiloxy) ethane synthesized in Step 3 of Example 1 was added thereto. After the reaction mixture was stirred at 90°C for 2 hours, a saturated ammonium chloride aqueous solution was added, and extracted three times with ethyl acetate. The combined ethyl acetate layers were dried over anhydrous sodium sulfate,

and concentrated under reduced pressure. The resulting crude product was purified by silica gel column chromatography (elution solvent: hexane/ethyl acetate = 10/1) to obtain 499 mg (corresponding to 1.19 mmol) of 4/-(2"-t-butyldiphenylsiloxyethoxy)acetophenone (Fig. 7, Step 1).

10 mL of ethyl acetate was added to 585 mg (corresponding to 2.62 mmol) of cupric bromide to obtain a suspension, to which 499 mg (corresponding to 1.19 mmol) of 4'-(2"-t-butyldiphenylsiloxyethoxy)acetophenone was added. Then, the resulting mixture was heated under reflux. After 2 hours, the reaction mixture was cooled down to room temperature and filtered. The resulting filtrate was concentrated under reduced pressure. The residue was dissolved in ethyl acetate and subjected to decoloring operation with addition of active charcoal. Then, the resulting solution was filtered and concentrated. The resulting crude product was purified by flash silica gel column chromatography (elution solvent: hexane/ethyl acetate = 10/1) to obtain 398 mg (corresponding to 0.800 mmol) of 2-bromo-4'-(2"-t-butyldiphenylsiloxyethoxy)acetophenone (Fig. 7, step 2).

398 mg (corresponding to 0.800 mmol) of 2-bromo-4'-(2"-t-butylediphenylsiloxyethoxy)acetophenone and 194 mg (corresponding to 0.880 mmol) of 2-amino-5-iodopyridine were dissolved in 5.0 mL of acetonitrile. The resulting solution was heated under reflux in an oil bath at 110°C for 4 hours. After the completion of the reaction, the reaction solution was cooled down to room temperature, and precipitates were filtered and recovered. The precipitates were washed with acetonitrile and dried under reduced pressure, to obtain 271 mg (corresponding to 0.386 mmol) of 2-[4'-(2"-t-butyldiphenylsiloxyethoxy)phenyl]-6-iodoimidazo[1,2-a]pyridine·hydrogenbromide (Fig. 7, Step 3).

180 mg (corresponding to 0.257 mmol) of 2-[4'-(2"-t-butyldiphenylsiloxyethoxy)phenyl]-6-iodoimidazo[1,2-a]pyridine·hydrogenbrominde was dissolved in 3.0 mL of acetonitrile, and 36 μL (corresponding to 0.257 mmol) of triethylamine was added thereto. Then, the reaction mixture was cooled to 0°C, and 48 μL (corresponding to 0.514 mmol) of chlorosulfonylisocyanate was dropped slowly. After the reaction mixture was stirred at room temperature for 3 days, a saturated aqueous sodium hydrogencarbonate solution was added, and extracted three times with dichloromethane. The combined dichloromethane layers were dried over anhydrous sodium sulfate, and concentrated under reduced pressure. The resulting crude product was purified by silica gel column chromatography (elation solvent: hexane/ethyl acetate = 5/1) to obtain 143 mg (corresponding to 0.222 mmol) of 2-[4'-(2"-t-butyldiphenylsiloxyethoxy)phenyl]-3-cyano-6-iodoimidazo[1,2-a]pyridine (Fig. 7, Step 4).

143 mg (corresponding so 0.222 mmol) of 2-[4'-(2"-t-butyldiphenylsiloxyethoxy)phenyl]-3-cyario-6-iodoimidazo[1,2-a]pyridine was dissolved in 1.0 mL of tetrahydrofuran, and 0.23 mL of a 1.0 mol/L tetrahydrofuran solution of tetrabutylammoniumfluoride was added thereto. After the reaction mixture was stirred at room temperature for 30 minutes, ammonium chloride aqueous solution was added followed by addition off 5.0 mL of water and 1.0 mL of acetonitrile to filter precipitates. The filtered precipitates were washed with wafer and acetonitrile in this order so obtain 84.0 mg (corresponding to 0.207 mmol) of 3-cyano-2-[4'-(2"-hydroxyethoxy)phenyl]-6-iodoimidazo[1,2-a]pyridine (Fig. 7, Step 5).

The NMR measurement results of the resulting 3-cyano-2-[4'-(2"hydroxyethoxy)phenyl]-6-iodoimidazo[1,2-a]pyridine (internal standard: tetramehtylsilane) are shown below.

NMR apparatus employed: JNM-ECP-500 (manufactured by Japan Electron Optics Laboratory Co., Ltd. (JEOL)) $^1$H-NMR (solvent: dimethylformamide-d6; resonance frequency: 500 MHz): δ 8.60 (s, 1H), 8.10 (d, J = 8.7 Hz, 2H), 7.59 (d, J = 9.1 Hz, 1H), 7.51 (d, J = 9.1 Hz, 1H), 7.11 (d, J = 8.7 Hz, 2H), 4.95 (t, J = 5.5 Hz, 1H), 4.13 (t, J = 4.6 Hz, 2H), 3.85 (t, J = 4.6 Hz Hz, 2H).

Example 11: Synthesis of 6-tributylstannyl-3-cyano-2-[4' (2"-hydroxvethoxy)phenyl]imidazo[1,2-a]pyridine

50 mg (corresponding to 0.123 mmol) of 3-cyano-2-[4'-(2"-hydroxyethoxy)phehy]-6-iodoimidazo[1,2-a]pyridine obtained above in Example 10 was dissolved in 2.0 mL of dioxane, and 1.0 mL of triethylamine was added thereto. Then, 0.10 mL (corresponding to 0.19 mmol) of bis (tributyltin) and 8.5 mg (a catalytic amount) of tetrakis-triphenylphosphine palladium were added thereto. After the reaction mixture was stirred at 90°C for 20 hours, the solvent was distilled off under reduced pressure. The residue was purified by flash silica gel column chromatography (elution solvent: hexane/ethyl acetate = 1/2) to obtain 43.2 mg (corresponding to 0.0760 mmol) of 6-tributylstannyl-3-cyano-2-[4'-(2" hydroxyethoxy)phenyl]imidazo[1,2-a]pyridine (Fig. 8, Step 1).

The NMR measurement results of the resulting 6-tributylstannyl-3-cynao-2-[4'-(2"-hydroxyethoxy)phenyl]imidazo[1,2-a]pyridine (internal standard: tetramehtylsilane) are shown below.

NMR apparatus employed: JNM-ECP-500 (manufactured by Japan Electron Optics Laboratory Co., Ltd. (JEOL)) $^1$H-NMR (solvent: chloroform-d1; resonance frequency:

500 MHz): δ 8.08 (d, J = 8.7 Hz, 2H) 8.00 (s, 1H), 7.59 (d, J = 8.7 Hz, 1H), 7.24 (d, J = 8.7 Hz, 1H), 7.03 (d, J = 8.7 Hz, 2H), 4.15 (d, J = 4.6 Hz, 2H), 4.00 (t, J = 4.6 Hz, 2H), 1.63-1.50 (m, 6H), 1.41-1.33 (m, 6H), 1.22-1.12 (m, 6H), 0.92 (t, J = 7.4 Hz, 9H)

Example 12: Synthesis of 3-cyano-2- [4'-(2''-hydroxyethoxy)phenyl]-6-[$^{123}$I]iodoimidazo[1,2-a]pyridine

To 70 μL of a solution of 6-tributylstannyl-3-cyano--2-[4'-(2''-hydroxyethoxy)phenyl]imidazo[1,2-a]pyridine (concentration: 1mg/mL) in a mixed solution of methanol/dimethylsulfoxide = 9/1, 70 μL of 1 mol/L hydrochloric acid, 20 μL of 1 mmol/L sodium iodide, 100 μL of [$^{123}$I] sodium iodide of 6.4 MBq and 20 μL of 10 % (w/v) hydrogen peroxide were added. After the mixed solution was left to stand at 50°C for 10 minutes, it was subjected to HPLC under the following conditions to obtain a fraction of 3-cyano-2-[4'-(2''-hydroxyethoxy)phenyl]-6-[$^{123}$I]iodoimidazo[1,2-a]pyridine.

HPLC conditions:

Column: Phenomenex Luna C18 (trade name; manufactured by Phenomenex Co.; size : 4.6 x 150 mm)
Mobile phase: 0.1 % trifluoroacetic acid in water/0.1 % trifluoroacetic acid in acetonitrile = 20/80 to 0/100 (17 minutes)
Flow rate: 1.0 mL/min.
Detector: Ultraviolet visible absorptiometer (Detection wavelength: 282 nm) and radioactivity counter (manufactured by raytest: type STEYFI)

10 ml of water was added to the fraction. The resulting solution was passed through a reversed phase column (trade name: Sep-Pak (registered trademark) Light C8 Cartridges manufactured by Waters: the packed amount of the packing agent: 145 mg) so that the column adsorbs and collects 3-cyano-2-[4'-(2''-hydroxyethoxy)phenyl]-6-[$^{123}$I]iodoimidazo[1,2-a]pyridine. The column was rinsed with 1 mL of water, and then 1 mL of diethyl ether was passed therethrough to elute 3-cyano-2-[4'-(2''-hydroxyethoxy)phenyl]-6-[$^{123}$I]iodoimidazo[1,2-a]pyridine. The amount of radioactivity of the obtained compound was 499 MBq at the end of synthesis. Further, the TLC analysis was conducted under the following conditions, and as we result, the radiochemical purity of the compound was 94%.

TLC analysis conditions:

TLC plate: Silica Gel 60 $F_{254}$ (trade name; manufactured by Merck & Co., Inc.)
Mobile phase: Chloroform/methanol/triethylamine = 100/1/2 Detector: Rita Star (trade name; manufactured by raytest)

Example 13: Synthesis of 2-[4'-(2''-fluoroethoxy) phenyl]-3-iodo-6-methoxyimidazo[1,2-a]pyridine (non-radioactive iodinated form)

100.0 g (corresponding to 0.575 mol) of 2-bromo-3-hydroxypyridine was dissolved in 310 mL of dimethylsulfoxide, and 575 mL (corresponding to 0.575 mol) of a 1 mol/L sodium methoxide-methanol solution was added thereto. Then, the reaction solution was heated to 90°C to distill off methanol. After the reaction solution was cooled down to 10°C or lower, 93.9 g (corresponding to 0.662 mol) of methyl iodide was added, and then stirred at room temperature for 20.5 hours. After the completion of the reaction, the reaction solution was poured into ice water and extracted twice with chloroform. The combined chloroform layer was washed with a 1 mol/L sodium hydroxide solution, washed twice with a saturated sodium chloride solution, and dried over anhydrous sodium sulfate. After the solvent was distilled off under reduced pressure, 65.4 g (corresponding to 0.343 mol) of 2-bromo-3-methoxypyridine was obtained (Fig. 9, Step 1).

262 mL of conc. sulfuric acid was cooled down to -2°C, and 262 mL of 90 % nitric acid was carefully added thereto. Subsequently, 65.3 g (corresponding to 0.347 mmol) of 2-bromo-3-methoxypyridine was carefully added thereto. After the reaction mixture was stirred in an ice bath for 10 minutes, the mixture was stirred at room temperature for 30 minutes, and then was heated to 55°C and further stirred for 1.5 hours. After the reaction solution was cooled, the reaction solution was poured little by little into crushed ice to generate precipitates. The precipitates were filtered and washed with water, and then dried over phosphorous pentoxide under reduced pressure, to obtain 55.7 g (corresponding to 0.239 mmol) of 2-bromo-3-methoxy-6-nitropyridine (Fig. 9, Step 2).

55.6 g (corresponding to 0.239 mol) of 2-bromo-3-methoxy-6-nitropyridine was dissolved in 1700 mL of ethanol, and 37.3 g (50% wet) of 10 palladium-carbon was added thereto under argon stream. To the mixture, 283 mL of hydrazine monohydrate was then added dropwise. After the reaction mixture was heated under reflux for 70 minutes, the reaction solution was cooled down to room temperature. Then, after palladium-carbon was filtered off, the residue was washed with ethanol, and the washings were combined with the filtrate. The combined solution was concentrated under reduced pressure. Then, 1300 mL of water and 130 mL of conc. aqueous ammonia were added to the concentrate, and the resulting mixture was extracted eight times with chloroform. The combined chloroform layer was dried over anhydrous sodium sulfate and concentrated under reduced pressure. The resulting crude product was distilled under reduced pressure to obtain 26.2 g (corresponding to 0.211 mol) of 2-amino-5-methoxypyridine (Fig. 9, Step 3).

30 mL of ethyl acetate was added to 4.69 g (corresponding to 21.0 mmol) of cupric bromide to obtain a suspension, to which a solution of 1.36 g (corresponding to 10.0 mmol) of 4'-hydroxyacetophenone in 30 mL of ethyl acetate was added, and the resulting mixture was heated under reflux. After 1.5 hours, the reaction mixture was cooled

down to room temperature and filtered. The resulting filtrate was concentrated under reduced pressure. The residue was dissolved in ethyl acetate and subjected to decoloring operation with addition of active charcoal. Then, the resulting solution was filtered and concentrated. The resulting crude product was purified by flash silica gel column chromatography (elution solvent: chloroform/methanol = 20/1), to obtain 2.15 g (corresponding to 10.0 mmol) of 2-bromo-4'-hydroxyacetophenone (Fig. 9, Step 4).

645 mg (corresponding to 3.00 mmol) of 2-bromo-4'-hydroxyacetophenone and 372 mg (corresponding to 3.00 mmol) of 2-amino-5-methoxypyridine were dissolved in 20 mL of acetonitrile. The resulting solution was heated under reflux in an oil bath at 90°C for 2 hours. After the completion of the reaction, the reaction solution was cooled down to room temperature, and precipitates were filtered and recovered. The precipitates were washed with acetonitrile and dried under reduced pressure to obtain crude crystals. The resulting crude crystals were suspended in a mixed solution of 4.0 mL of water and 4.0 mL of methanol. Then, about 2.0 mL of a saturated sodium hydrogencarbonate solution was added thereto, and the mixture was sonicated for 5 minutes using an ultrasonic washing machine. Precipitates were filtered and recovered from the resulting mixture, sufficiently washed with water, and dried under reduced pressure, to obtain 512 mg (corresponding to 2.17 mmol) of 2-(4'-hydroxypheny)-6-methoxyimidazo[1,2-a]pyridine (Fig. 9, Step 5).

246 mg (corresponding to 1.02 mmol) of 2-(4'-hydroxyphenyl)-6-methoxyimidzo[1,2-a]pyridine that was sufficiently dried to remove moisture was dissolved in 9.0 mL of N,N-dimethylformamide, and 353 mg (corresponding to 3.06 mmol) of potassium carbonate was added thereto. The mixture was supplemented with 279 mg (corresponding to 1.53 mmol) of 2-fluoro-1-paratoluenesulfonyloxyethane, and then the solution was stirred at 90°C for 2 hours. After the completion of the reaction, a saturated aqueous ammonium chloride solution was added thereto, and extracted three times with ethyl acetate. The combined ethyl acetate layers were washed with water and a saturated sodium chloride solution, dried over anhydrous sodium sulfate, and concentrated under reduced pressure. The resulting crude product was purified by silica gel column chromatography (elution solvent: ethyl acetate), to obtain 247 mg (corresponding to 0.863 mmol) of 2-[4'-(2'-fluoroethoxy)phenyl]-6-methoxyimidazo[1,2-a]pyridine (Fig. 9, Step 6).

247 mg (corresponding to 0.863 mmol) on 2-[4'-(2''-fluoroethoxy)phenyl]-6-methoxyimidazo[1,2-a]pyridine was dissolved in 8.0 mL of acetonitrile, and 233 mg (corresponding to 1.04 mmol) of N-iodosnccineimide was added thereto. The reaction mixture was stirred at 0°C for 20 minutes, and then a saturated aqueous sodium hydrogencarbonate solution and a saturated aqueous sodium thiosulfate solution were added thereto, and extracted three times with ethyl acetate. The combined ethyl acetate layers were washed water and a saturated sodium chloride solution, dried over anhydrous sodium sulfate, and concentrated under reduced pressure. The resulting crude product was purified by silica gel column chromatography (elution solvent: hexane/ethyl acetate = 1/2), to obtain 3.52 mg (corresponding to 0.85 mmol) of 2-[4'-(2''-fluoroethoxy)phenyl]-3-iodo-6-methoxyimidazo[1,2-a]pyridine (Fig. 9, Step 7).

The NMR measurement results of the resulting 2-[4'-(2''-fluoroethoxy)phenyl]-3-iodo-6-methoxyimidazo[1,2-a]pyridine (internal standard: tetramehtylsilane) are shown below.

NHR apparatus employed: JNM-ECP-500 (manufactured by Japan Electron Optics Laboratory Co., Ltd. (JEOL)) $^1$H-NMR (solvent: chloroform-d1; resonance frequency: 500 MHz) :$\delta$ 7.99 (d, J = 8.7 Hz, 2H), 7.74 (s, $^1$H), 7.51 (d, J = 9.6 Hz, 1-H), 7.06-7.02 (m, 3H), 4.79 (dt, $^2J_{HF}$ = 48.0 Hz, J = 4.6 Hz, 2H), 4.29 (dt, $^3J_{HF}$ = 27.5 Hz, J = 4.6 Hz, 2H), 3.91 (s, 3H).

Example 14: Synthesis of 2-[4'-(2''-fluoroethoxy)phenyl]-3-[$^{123}$I]iodo-6-methoxyimidazo[1,2-a]pyridine

To 60 $\mu$L of a solution of 2-[4'-(2''-fluoroethoxy)phenyl]-6-methoxyimidazo[1,2-a]pyridine (concentration: 1 mg/mL) in a mixed solution of methanol/dimethylsulfoxide (in a ratio of 9/1), 70 $\mu$L of 2 mol/L hydrochloric acid, 15 $\mu$L of 1 mmol/L sodium iodide, 100 $\mu$L of [$^{123}$I] sodium iodide of 243 MBq and 15 $\mu$L of 10 % (w/v) hydrogen peroxide were added. After the mixed solution was left to stand at 50°C for 10 minutes, the solution was subjected to HPLC under the following conditions, to obtain a fraction of 2-[4'-(2''-fluoroethoxy)phenyl]-3-[$^{123}$I]iodo-6-methoxyimidazo[1,2-a]pyridine.

HPLC conditions:

Column: Phenomenex Luna C18 (trade name; manufactured by Phenomenex Co.; size: 4.6 x 150 mm)
Mobile phase: 0.1. % trifluoroacetic acid in water/0.1 % trifluoroacetic acid in acetonitrile = 20/80 to 0/100 (17 minutes)
Flow rate: 1.0 mL/min.
Detector: Ultraviolet visible absorptiometer (Detection wavelength: 282 nm) and radioactivity counter (manufactured by type STEFFI)

10 ml of water was added to the fraction. The resulting solution was passed through a reversed phase column (trade name: Sep-Pak (registered trademark) Light C8 Cartridges manufactured by Wasters: the packed amount of the packing agent: 145 mg) so that the column adsorbs and collects 2-[4'-(2''-fluoroethoxy)phenyl]-3'-[$^{123}$I]iodo-6-methoxyimidazo[1,2-a]pyridine. The column was rinsed with 1 mL of water, and then 1 mL of diethyl ether was passed therethrough to elute 2-[4'-(2''-fluoroethoxy)phenyl-3-[$^{123}$I]iodo-6-methoxyimidazo[1,2-a]pyridine. The amount of radioactivity

of the obtained compound was 96.9 MBq at the end of synthesis. Further, the TLC analysis was conducted under the following conditions, and as a result, the radiochemical purity of the compound was 89.6%.

TLC analysis conditions:

TLC plate: Silica Gel 60 $F_{234}$ (trade name; manufactured by Merck & Co., Inc.)
Mobile phase: Chloroform/methanol/triethylamine = 100/1/2
Detector: Rita Star (trade name; manufactured by raytest)

<u>Reference Example 1: Synthesis of [$^{123}$I]-IMPY</u>

[$^{123}$-I]-TMPY was synthesized in accordance with the following steps for use in Comparative Examples for evaluations on measurement of $\log P_{octanol}$.

In accordance with the method described in a literature (Zhi-Ping Zhuang et al., J. Med. Chem, 2003, 46, p. 237-243), 6-tributylstannyl-2-[4'-(N,N-dimethylamino)phenyl]imidazo[1,2-a]pyridine was synthesized, and dissolved in methanol (concentration: 1mg/mL). To 53 μL of the resulting solution, 75 uL of 1 mol/L hydrochloric acid, 60-70 μL of [$^{123}$I]sodium iodide
of 224-253 MBq, 10 μL of a 1 mmol/L sodium, iodide solution and 15 μL of 10% (w/v) hydrogen peroxide were added. After the mixed solution was left to stand at 50°C for 10 minutes, the solution was subjected to HPLC under the same conditions as in Example 3, to obtain a fraction of [$^{123}$I]-IMPY.

10 ml of water was added to the fraction. The resulting solution was passed through a reversed phase column (trade name: Sep-Pak (registered trademark) Light C8 Cartridges manufactured by Wasters; the packed amount of the packing agent: 145 mg), so that the column adsorbs and collects the [$^{123}$]-IMPY. The column was rinsed with 1 mL of water, and then 1 mL of diethyl ether was passed therethrough, to elute [$^{123}$I]-IMPY. The obtained radioactivity was 41-57 MBq at the end of synthesis. Further, the TLC analysis was conducted under the same conditions as described in Example 3, and as a result, the radiochemical purity of the compound was 93%.

<u>Examples 15 to 18, Comparative Example 1: Measurement of partition coefficient based on the octanol extraction method</u>

Partition coefficients based on the octanol 1 extraction method (hereinafter referred to as $\log P_{octanol}$) were measured, which are generally known as an indicator of permeability of compounds through the blood-brain barrier (hereinafter referred to as BBB).

<u>Method</u>

Each diethyl ether solution of Compound 1, Compound 2, Compound 3 and Compound 4 as well has a diethyl ether solution of [$^{123}$I]-IMPY were each diluted with 10 mg/mL ascorbic acid-containing physiological saline solution, and adjusted to a radioactive concentration of 27-30 MBq/mL. To 2 mL of octanol, 10 μL each of these solutions was added, and 2 mL of 10 mmol/L phosphate buffer (pH 7.4) was added, followed by stirring for 30 seconds. After each mixture was centrifuged (2000 rpm x 60 min.) with a low-speed centrifuge (type: CTD4, manufactured by Hitachi Koki Co., Ltd), the octanol layer and the water layer were sampled each in an amount of 1 mL, and subjected to measurement of radioactivity count with an autowell gamma system (Type: ARC-301B, manufactured by Aloka). Using the obtained radioactivity count, was calculated in accordance with the equation (1).

$$\log P_{octanol} = \log_{10}\left(\frac{\text{Radioactivity count of octanol layer}}{\text{Radioactivity count of water layer}}\right) \cdots (1)$$

<u>Results</u>

The results are shown in Table 2. As shown in the Table, all the compounds showed 10 values between 1 and 3. It is known that compounds permeable to BBB show a $\log P_{octanol}$ value between 1 and 3 (Douglas D. Dischino et al., J. Nucl. Med., (1983), 24, p.1030-1038). From the above results, it is implied that each of the compounds used in the present experiment has a BBB permeability like IMPY.

Table 2: $logP_{octanol}$ value of the present compound

| Experiment | Compound | $logP_{octanol}$ value |
|---|---|---|
| Comparative Example 1 | [123I]-IMPY | 1.9 |
| Example 15 | Compound 1 | 2.3 |
| Example 16 | Compound 2 | 2.2 |
| Example 17 | Compound 3 | 2.5 |
| Example 18 | Compound 4 | 3.0 |

Examples 19 to 2 Comparative Example 2: Measurement of transferability into brain and clearance

Using Compound 1, Compound 2, Compound 3 and Compound 4, a time course charge of radioactive accumulation in brain of male Wistar rats (7-week old) was measured.

Method

Compound 1 in 10 mg/mL ascorbic acid-containing physiological saline solution (27 MBq/mL in radioactive concentration). Compound 2 in 10 mg/mL ascorbic acid-containing physiological saline solution (27 MBq/mL in radioactive concentration). Compound 3 in 10 mg/mL ascorbic acid-containing physiological saline solution (28 MBq/mL in radioactive concentration) and Compound 4 in 10 mg/mL ascorbic acid-containing physiological saline solution (30 MBq/mL in radioactive concentration) were prepared to obtain sample solutions. The sample solutions were injected under thiopental anesthesia into the tail vein of the male Wistar rats (7-week old) (dosage: 0.05 mL, dosed radioactivity: 1.4-1.5 MBq equivalent). The rats were sacrificed by bleeding from abdominal artery, and brains were removed and subjected to measurement of mass of brains and further subjected to measurement of radioactivity (hereinafter referred to as A in this Example) with a single channel analyzer (detector type: SP-20 manufactured by OHYO KOKEN KOGYO Co,, Ltd.) 2, 5, 30 and 60 minutes after the injection. Further, the radioactivity level of the rest of the whole body was measured in the same manner as above (hereinafter referred to as B in this Example). Using these measurement results, radioactive accumulation per unit weight of brain (%ID/g) at the respective time points of dissection was calculated in accordance with the following formula (2) (Examples 19 to 22).
Separately, [123I]-IMPY in 10 mg/mL ascorbic acid-containing physiological saline solution (16 MBq/mL in radioactive concentration) was prepared, the same procedure as above was conducted, and radioactive accumulation per unit weight of brain (%ID/g) at the respective time points of dissection was calculated (Comparative Example 1).
Meanwhile, three animals were used for all Examples and Comparative Example 2 at the respective time points.
$$\%ID/\mathrm{g} = \frac{\mathrm{A}}{\mathrm{B} \times \mathrm{brain\ weight}} \times 100 \cdots (2)$$

Results

The results are shown in Table 3. As shown in Table 3, each compounds of the present invention showed a significant radioactive accumulation like [123I]-TMPY at the time point of two minutes after the injection, and then showed a tendency to rapidly clear away in 60 minutes. These results suggest that Compounds 1, 2, 3 and 4 possess excellent transferability to brain and rapid clearance from brain like [123I]-IMPY.
Table 3: Radioactive accumulation in brain of the present compound after intravenous injection (rats)

| Example | Compound | Radioactive accumulation per unit weight (%ID/g) | | | |
|---|---|---|---|---|---|
| | | After 2 min. | After 5 min. | After 30 min. | After 60 min. |
| Example 19 | Compound 1 | 0.91 | 0.60 | 0.08 | 0.03 |

(continued)

| Example | Compound | Radioactive accumulation per unit weight (%ID/g) | | | |
|---|---|---|---|---|---|
| | | After 2 min. | After 5 min. | After 30 min. | After 60 min. |
| Example 20 | Compound 2 | 0.57 | 0.36 | 0.05 | 0.01 |
| Example 21 | Compound 3 | 0.50 | 0.34 | 0.05 | 0.01 |
| Example 22 | Compound 4 | 0.32 | 0.67 | 0.18 | 0.04 |
| Comparative Example 2 | [$^{123}$I]-IMPY | 1.19 | 0.97 | 0.23 | 0.09 |

Examples 23 to 26: Confirmation of imaging of amyloid in brain

The following experiment was carried out in order to examine whether amyloid in brain can be imaged by the compound of the present invention.

Method

(1) A$\beta_{1-42}$ (manufactured by Wako) was dissolved in phosphate buffer (pH 7.4) and shaken at 37˚C for 72 hours, to obtain a 1 mg/mL suspension aggregates Aβ (hereinafter referred to as amyloid suspension in the Examples).

(2) Under thiopental anesthesia, 2.5 μL (corresponding to 25 μg) of the amyloid suspension was injected into an amygdaloid nucleus on one side of a male Wistar rat (7-week old). As a control, 2.5 μL of a phosphate buffered physiological saline solution (pH 7.4) was injected into an amygdaloid nucleus on the other side of the rat. The rats were examined 1 day after the injection of the amyloid suspension and the phosphate buffered physiological saline solution (pH 7.4).

(3) Compound 1 in a 10 mg/mL ascorbic acid-containing physiological saline solution (27 MBq/mL in radioactive concentration, Example 23), Compound 2 in a 10 mg/mL ascorbic acid-containing physiological saltine solution (27 MBq/mL in radioactive concentration, Example 24), Compound 3 in a 10 mg/mL ascorbic acid-containing physiological saline solution (28 MBq/mL in radioactive concentration, Example 25) and Compound 4 in a 10 mq/mL ascorbic acid-containing physiological saline solution (30 MBq/mL in radioactive concentration, Example 26) were each prepared to obtain a sample solution. This sample solution was injected under thiopental anesthesia into the rat through the tail vein (dosage: 0.5 mL, dosed radioactivity: 14-15 MBq equivalent.).

(4) Braid was removed 60 minutes after the injection to prepare a brain slice of 10 μm in thickness with a microtome (type: CM3050S, manufactured by LEICA). The brain slice was exposed to an imaging plate for 20 hours, and then image analysts was carried out by use of a Bio-imaging Analyzer (type: BAS-2500; manufactured by FUJI-FILM Corporation).

(5) After the completion of the image analysis using the Bio-imaging Analyzer, pathological staining with Thioflavin T was carried out to perform imaging by use of a fluorescence microscope (manufactured by NIKON Corporation; type: TE2000-U model; excitation wavelength: 400-440 nm; detection wavelength: 470 nm). Thus, it was confirmed that amyloid was deposited on the slice (Fig. 10b, Fig. 11b, Fig. 12b and Fig. 13).

Results

Figs. 10-13 show images by autoradiogram and Thioflavin T staining of the brain slice of the rat to which amyloid was injected intracerebrally. As shown in this figure, a marked accumulation of radioactivity was observed in the amygdaloid nucleus on the side to which the amyloid suspension was injected. From the result of Thioflavin T staining in the site where radioactivity was accumulated, it was confirmed that amyloid was present in the site. On the other hand, no significant accumulation of radioactivity was observed in the amygdaloid nucleus or the side to which the physiological saline solution was injected, compared with the other sites.
These results suggest that each compound has a property of accumulating at intracerebral amyloid and has a capability of imaging intracerebral amyloid.

Example 27: Reverse mutation test

In order to examine gene mutagenicity of Compound 5, reverse mutation test using *Salmonella typhimurium* TA98 and TA100 (hereinafter referred to as Ames test) was conducted.
The test was conducted without addition of S9mix and with addition of S9mix. Dimethylsulfoxide (hereinafter referred to as DMSO) was used as a negative control. A positive control was 2-(2-furyl)-3-(5-nitro-2-furyl) acrylamide (hereinafter referred to as AF-2) in case S9mix was not added, and 2-aminoanthracene (hereinafter referred to as 2-AA) in case S9mix was added.
As a sample solution to be added to a test plate, a solution at maximum concentration in which Compound 5 was dissolved in DMSO (concentration of compound: 50 mg/mL) and a sample solution to be added to a test plate at each concentration in which a solution at maximum concentration was diluted with DMSO were prepared. As a sample solution for preparing a sample of positive control, when TA98 was used as a test strain without addition of S9mix, a solution of AF-2 (compound concentration: 1 μg/mL) in DMSO was prepared; when TA100 was used a a test strain without addition of S9mix, a solution of AF-2 (compound concentration: 0.1 μg/mL) in DMSO was prepared; when TA98 was used as a test strain with addition of S9mix, a solution of 2-AA (compound concentration: 5 μg/mL) in DMSO was prepared; and when TA100 was used as a test strain with addition of S9mix, a solution of 2-AA (compound concentration: 10 μg/mL) in DMSO was prepared. The addition amount of each sample was made to he 0.1 mL/plate, and for a negative control, only DMSO was added so as to make it to be 0.1 mL/plate.
The results are shown in Table 4. The number of reverse mutation colonies in the group treated with Compound 2 was less than two times the group treated with the negative control, regardless of addition of S9mix and the addition amount of a sample to be tested. On the other hand, the group treated with the positive control showed obvious increase in the number of reverse mutation colonies. From the aforementioned results, it is judged that Compound 5 is negative in the Ames test and has no mutagenicity.
Table 4: Results of Ames test

| | Compound | Mutagenicity | | | |
|---|---|---|---|---|---|
| | | Without addition of S9mix | | With addition of S9mix | |
| | | TA98 | TA100 | TA98 | TA100 |
| Example 27 | Compound 5 | Negative | Negative | Negative | Negative |

INDUSTRIAL APPLICABILITY

The compound and detecting reagent of the present invention can bye utilized in the field of diagnostic agents.

BRIEF DESCRIPTION OF THE DRAWINGS

Fig. 1 is a scheme of synthesis of 2-[4'-(2''-hydroxyethoxy)phenyl]-6-iodo-3-methylimidazo[1,2-a]pyridine (non-radioactive iodinated form).
Fig. 2 is a scheme of synthesis of 6-tributylstannyl-2-[4'-(2''-hydroxyethoxy)phenyl]-3-methylimidazo[1,2-a]pyridine,
Fig. 3 is a scheme of synthesis of 2-(4'-fluorophenyl)-6-iodo-3-methylimidazo[1,2-a]pyridine (non-radioactive iodinated form).

Fig. 4 is a scheme of synthesis of 6-tributylstannyl-2-(4'-flucrophenyl)-3-methylimidazo[1,2-a]pyridine.
Fig. 5 is a scheme of synthesis of 2-(4'-hydroxyphenyl)-6-iodo-3-methylimidazo[1,2-a]pyridine (non-radioactive iodinated form).
Fig. 6 is a scheme of synthesis of 6-tributylstannyl-2-(4'-hydroxyphenyl)-3-methylimidazo[1,2-a]pyridine.
Fig. 7 is a scheme of synthesis of 3-cyano-2-[4'-(2"-hydroxyethoxy) phenyl]-6-iodoimidazo[1,2-a]pyridine (non-radioactive iodinated form).
Fig. 8 is a scheme of synthesis of 6-tributylstannyl-3-cyano-2-[4'-(2"-hydroxyethoxy)phenyl]-imidazo[1,2-a]pyridine.
Fig. 9 is a scheme of synthesis of 2-[4'-(2"-fluoroethoxy)phenyl]-3-iodo-6-methoxyimidazo[1,2-a]pyridine (non-radioactive iodinated form).
Fig. 10(a) is an autoradiogram of the brain slice after the injection of 2-(4'-fluorophenyl)-6-[$^{123}$I]iodo-3-methylimidazo [1,2-2]pyridine, and Fig. 10 (b) is a fluorescent microscopic image of the Thioflavin T stained sample (a magnification of the site to which the amyloid suspension was injected).
Fig. 11 (a) its an autoradiogram the brain slice after the injection of 2-[4'-(2"-hydroxyethoxy)phenyl]-6 [$^{123}$I]iodo-3-methylimidazo[1,2-a]pyridine, and Fig. 11(b) is a fluorescent microscopic image of the Thioflavin T stained sample (a magnification of the site to which the amyloid suspension was injected).
Fig. 12(a) is an autoradiogram of the brain slice after the injection of 2-(4'-hydroxyphenyl)-6-[$^{123}$I]iodo-3-methylimidazo[1,2-a]pyridine, and Fig. 12 (b) is a fluorescent microscopic image of the Thioflavin T stained sample (a magnification of the site to which the amyloid suspension was injected).
Fig. 13(a) is an autoradiogram of the brain slice after the injection of 3-cyano-2-[4'-(2"-hydroxyethoxy)phenyl]-6-[$^{123}$I] iodoimidazo[1,2-a]pyridine, and Fig. 13(b) is a fluorescent microscopic image of the Thioflavin T stained sample (a magnification of the site to which the amyloid suspension was injected).

## Claims

**1.** A compound represented by the following formula (1), or a salt thereof:

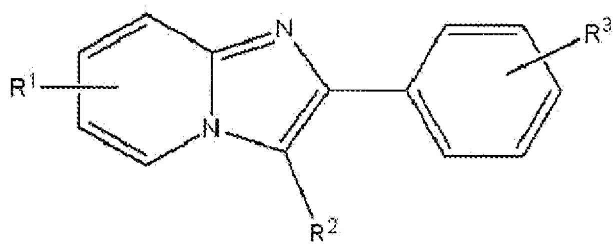

(1)

wherein $R^1$ is a group selected from the group consisting of a halogen substituent, an alkyl substituent with 1 to 4 carbon atoms, an alkoxy substituent having an alkyl chain with 1 to 4 carbon atoms and a hydroxyl group,
$R^2$ is a group selected from the group consisting of a cyano substituent, an alkyl substituent with 1 to 4 carbon atoms and a halogen substituent, and
$R^3$ is a group selected from the group consisting of a hydroxyl group, a halogen substituent and a substituent represented by the following formula (2):

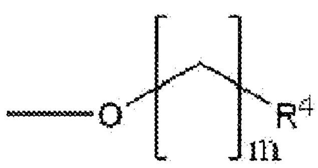

(2)

wherein $R^4$ is a hydrogen, a halogen substituent or a hydroxyl group, and m is an integer of 1 to 4,

provided that at least one of $R^1$, $R^2$, $R^3$ and $R^4$ represents a radioactive halogen.

2. A compound or a salt thereof according to claim 1, wherein at least one of $R^1$, $R^2$, $R^3$ and $R^4$ represent a radioactive halogen selected from the group consisting of $^{18}F$, $^{76}Br$, $^{123}I$, $^{124}I$, $^{125}I$ or $^{131}I$.

3. A compound or a salt thereof according to clam 2, which is selected from the group consisting of 2-(4'-fluorophenyl)-6-[$^{123}$I]iodo-3-methylimidazo[1,2-a]pyridine, 2-[4'-(2"-hydroxyethoxy)phenyl]-6-[$^{123}$I]icdo-3-methylimidazo[1,2-a]pyridine, 2-(4'-hydroxyphenyl)-6-[$^{123}$I]iodo-3-methylimidazo[1,2-a]pyridine, 3-cyano-2-[4'-(2"-hydroxyethoxy)phenyl]-6-[$^{123}$I]iodoimidazo[1,2-a]pyridine, 2-[4'-(2"-[$^{18}$F]fluoroethoxy)phenyl]-3-iodo-6-methoxyimidazo[1,2-a]pyridine, 2-[4'-(2"-[$^{18}$F]fluoroethoxy)phenyl]-6-methoxy-3-methylimidazo[1,2-a]pyridine and 2-(4'[$^{18}$F]fluorophenyl)-6-iode-3-methylimidazo[1,2-a]pyridine.

4. A compound represented by the following formula (3), or a salt thereof:

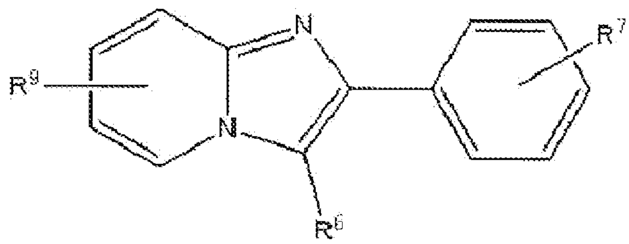

(3)

wherein $R^3$ is a group selected from the group consisting of a non-radioactive halogen substituent, a nitro group, a trialkylstannyl substituent having alkyl chains with 1 to 4 carbon atoms or a triphenylstannyl group,
$R^6$ is a group selected, from the group consisting of a cyano substituent, an alkyl substituent with 1 to 4 carbon atoms and a halogen substituent, and
$R^7$ is a group selected from the group consisting of a hydroxyl group, a halogen substituent and a substituent represented by the following formula (4):

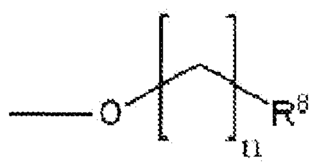

(4)

wherein $R^8$ is a hydrogen, a halogen substituent or a hydroxyl groups and m is an integer of 1 to 4.

5. A compound represented by the following formula (5), or a salt thereof:

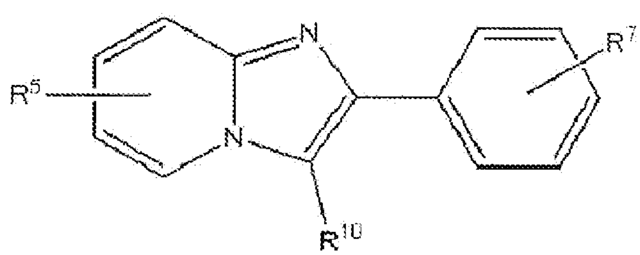

(5)

wherein $R^5$ is a group selected from the group consisting of a halogen substituent, an alkyl substituent with 1 to 4 carbon atoms, an alkoxy substituent having an alkyl chain with 1 to 4 carbon atoms and a hydroxyl group, $R^{10}$ is a group selected from the group consisting of a non-radioactive halogen substituent, a nitro group, a trialkylstannyl substituent having alkyl chains with 1 to 4 carbon atoms or a triphenylstannyl group, and
$R^7$ is a group selected from the group consisting of a hydroxyl group, to halogen substituent and a substituent represented by the following formula (4):

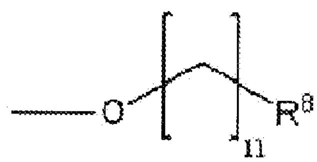

(4)

wherein $R^8$ is a hydrogen, a halogen substituent or a hydroxyl group, and n is an integer of 1 to 4.

**6.** A compound represented by the following formula (6), or a salt thereof:

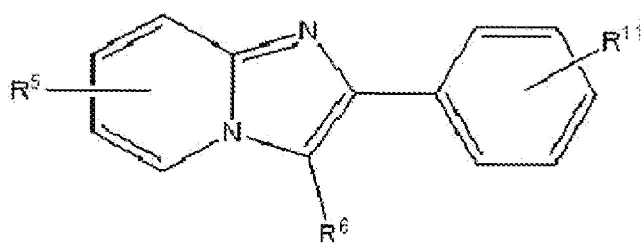

(6)

wherein $R^5$ is a group selected from the group consisting of a halogen substituent, an alkyl substituent with 1 to 4 carbon atoms, an alkoxy substituent having an alkyl chain with 1 to 4 carbon atoms and a. hydroxyl group,
$R^6$ is a group selected from the group consisting of a cyano substituent, an alkyl substituent with 1 to 4 carbon atoms and a halogen substituent, and
$R^{11}$ is a group selected from the group consisting of a non-radioactive halogen substituent, a nitro substituent, et trialkylstannyl substituent having alkyl chains with 1 to 4 carbon atoms and a triphenylstannyl group.

**7.** A compound represented by the following formula (7), or a salt thereof:

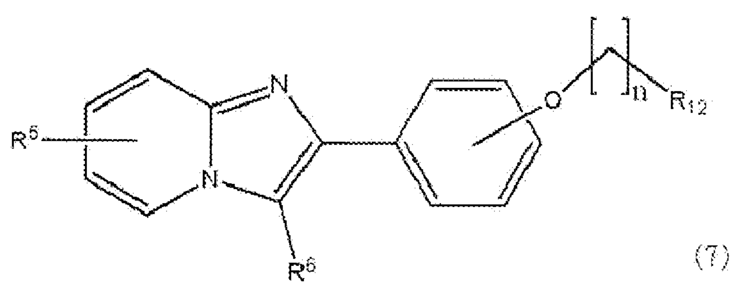

(7)

wherein $R^5$ is a group selected from the group consisting of a halogen substituent, an alkyl substituent with 1 to 4 carbon atoms, an alkoxy substituent having an alkyl chain with 1 to 4 carbon atoms and a hydroxyl group, $R^6$ is a group selected from the group consisting of a cyano substituent, an alkyl substituent with 1 to 4 carbon atoms and a halogen substituent, and
$R^{12}$ is a group selected from the group consisting of a non-radioactive halogen substituent, a methanesulfonyloxy

substituent, a trifluoromethanesulfonyloxy substituent and an aromatic sulfonyloxy substituent.

**8.** A reagent for detecting amyloid deposited on a biological tissue, which comprises a compound represented by the following formula (1), or a salt thereof:

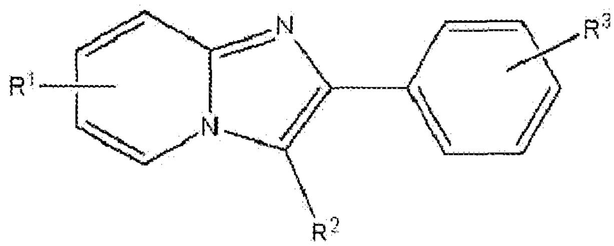

(1)

wherein $R^1$ is a group selected from the group consisting of a halogen substituent, an alkyl substituent with 1 to 4 carbon atoms, an alkoxy substituent having an alkyl chain with 1 to 4 carbon atoms and a hydroxyl group, $R^2$ is a group selected from the group consisting of a cyano substituent, an alkyl substituent with 1 to 4 carbon atoms and a halogen substituent, and
$R^3$ is a group selected from the group consisting of a a hydroxyl group, a halogen substituent and a substituent represented by the following formula (2):

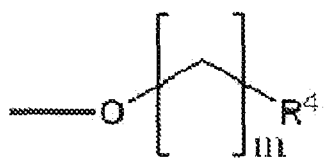

(2)

wherein $R^4$ is a hydrogen, a halogen substituent or a hydroxyl group, and m is an integer of 1 to 4,
provided that at least one of $R^1$, $R^2$, $R^3$ and $R^4$ represents a radioactive halogen.

**9.** A reagent for detecting amyloid deposited on a biological tissue, according to claim 8, wherein at least one of $R^1$, $R^2$, $R^3$ and $R^4$ represent a radioactive halogen selected from the group consisting of $^{18}F$, $^{76}Br$, $^{123}I$, $^{124}I$, $^{125}I$ or $^{131}I$.

**10.** A reagent for detecting amyloid deposited on a biological tissue, which comprises a compound selected from the group consisting of 2-(4'-fluorophenyl)-6-[$^{123}$I]iodo-3-methylimidazo[1,2-a]pyridine, 2-[4'-(2"-hydroxyethoxy)phenyl]-6-[$^{123}$I]iodo-3-methylimidazo[1,2-a]pyridine, 2-(4'-hydrcxyphenyl)-6-[$^{123}$I]iodo-3-methylimidazo-[1,2-a]pyridine, 3-cyano-2-[4'-(2"-hydroxyethoyx)phenyl]-6-[$^{123}$I]iodoimidazo[1,2-a]pyridine, 2-[4'-(2"-[$^{18}$F]fluoroethoxy)phenyl]-3-iodo-6-methoxyimidazo[1,2-a]pyridine, 2-[4'-(2"-[$^{18}$F]fluoroethoyx)phenyl]-6-methoxy-3-methylimidazo[1,2-a]pyridine and 2-(4'[$^{18}$F]fluorophenyl)-6-iodo-3-methylimidazo[1,2-a]pyridine.

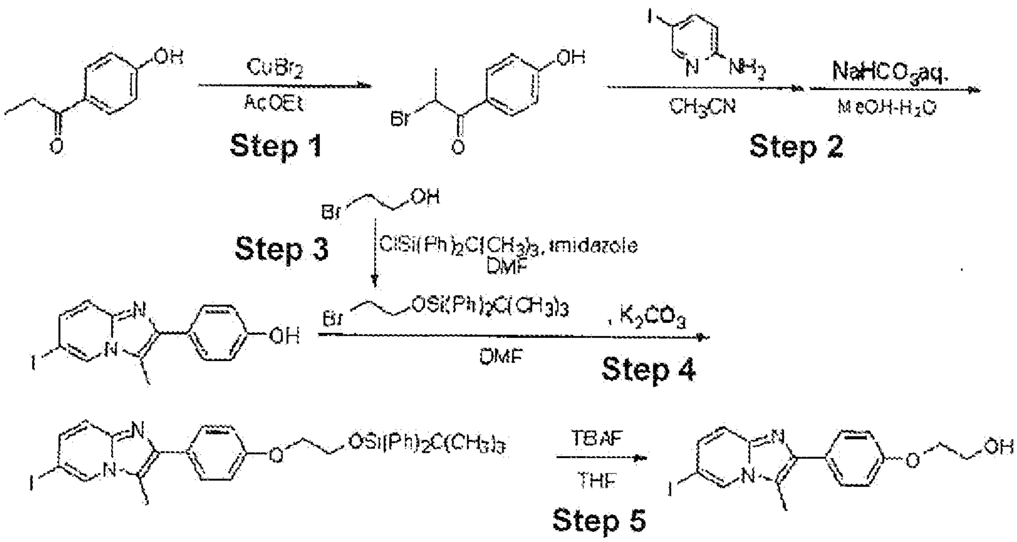


Figure 1

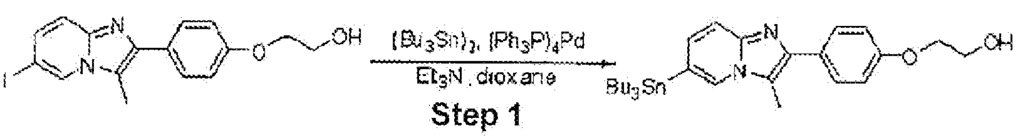


Figure 2

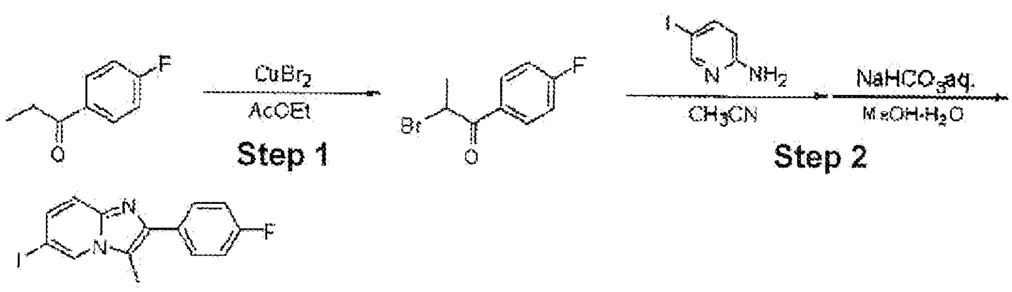


Figure 3

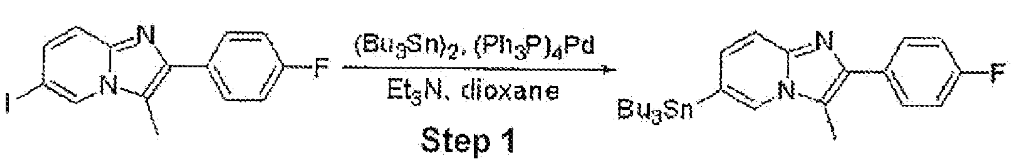


Figure 4

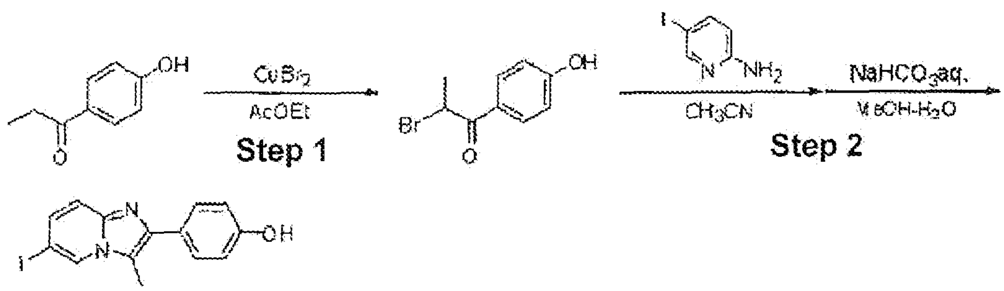


Figure 5

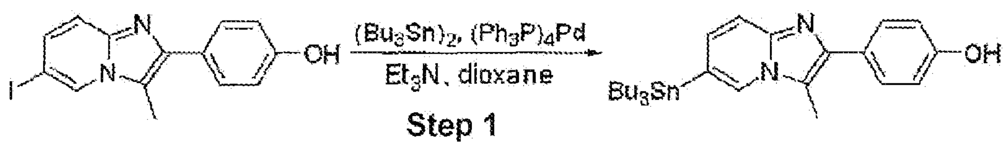


Figure 6

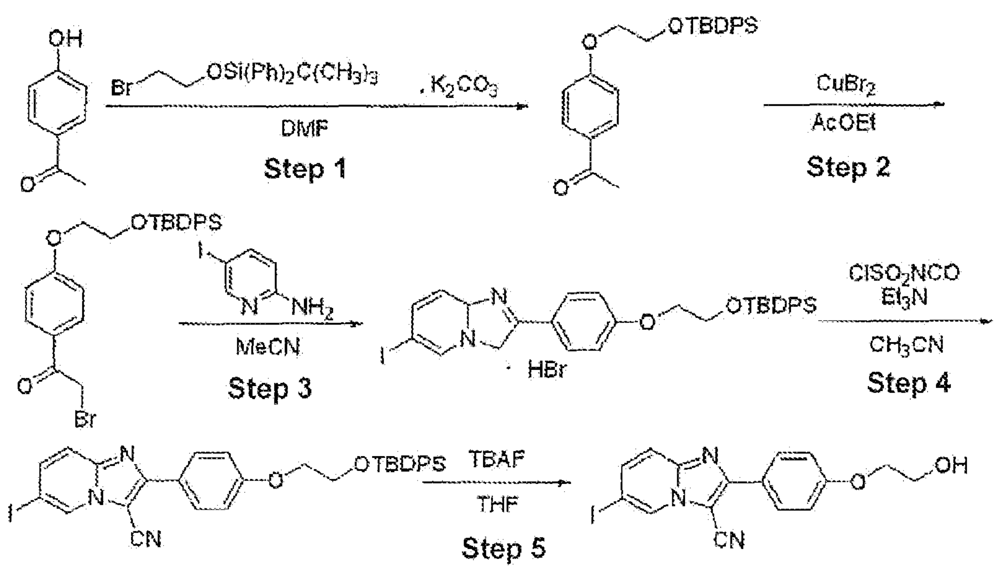


Figure 7

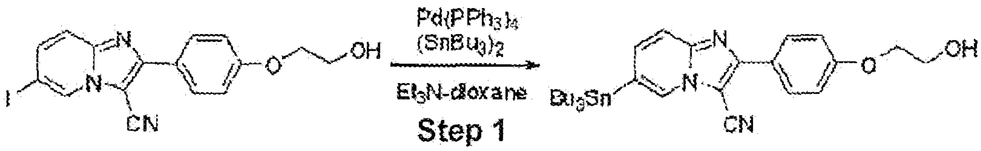


Figure 8

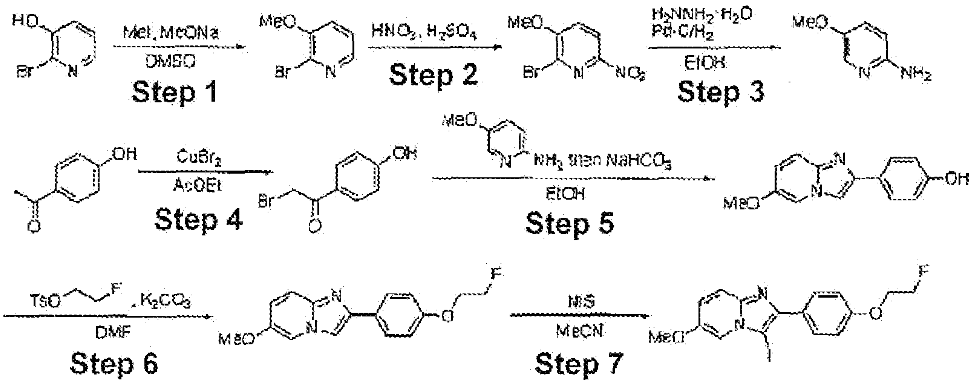


Figure 9

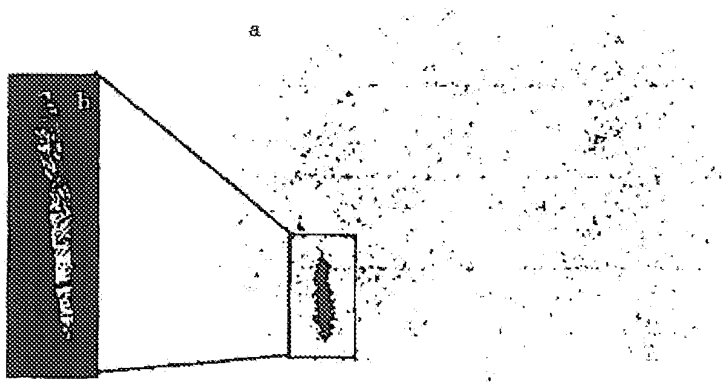


Figure 10

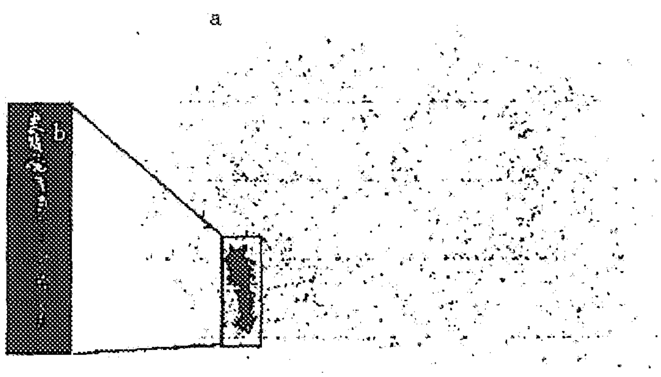


Figure 11

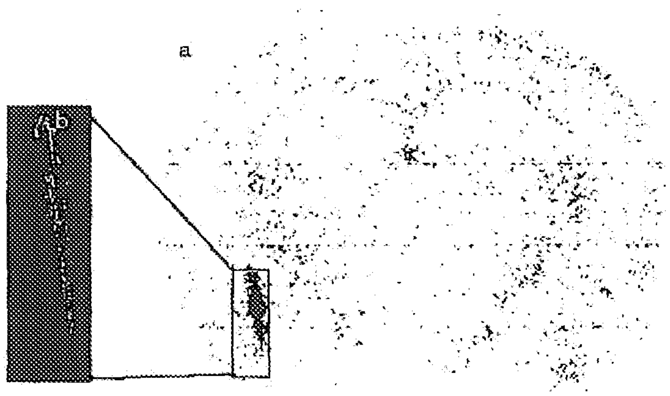


Figure 12

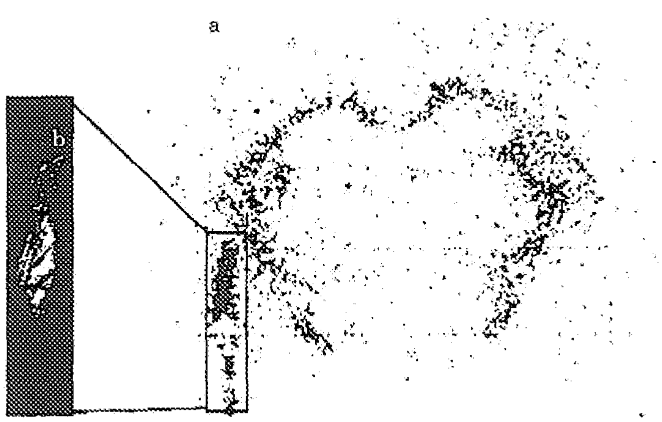


Figure 13

## INTERNATIONAL SEARCH REPORT

International application No. PCT/JP2008/069335

A. CLASSIFICATION OF SUBJECT MATTER
*C07D471/04*(2006.01)i, *A61K49/00*(2006.01)i, *A61K49/04*(2006.01)i

According to International Patent Classification (IPC) or to both national classification and IPC

B. FIELDS SEARCHED

Minimum documentation searched (classification system followed by classification symbols)
C07D471/04, A61K49/00, A61K49/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
CAplus(STN), REGISTRY(STN), MEDLINE(STN), BIOSIS(STN), EMBASE(STN), WPIDS(STN), JSTPlus(JDreamII), JMEDPlus(JDreamII), JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X<br>A | KRISTJAN, S. et al., Imidazo[1,2-a]pyridines with potent activity against herpesviruses, Bioorganic & Medicinal Chemistry Letters, 2007.05.15, Vol.17, p.2735-2739 | 4-6<br>1-3,7-10 |
| X<br>A | JP 2005-500315 A (SmithKline Beecham Corp.), 06 January, 2005 (06.01.05), Full text; particularly, scheme 4, compound XV; Par. No. [0258]<br>& US 7186714 B2 & WO 2003/000689 A1<br>& EP 1401836 A1 & BR 210464 A<br>& HU 400266 A & NZ 529568 A<br>& CZ 20033518 A & PL 366827 A<br>& CN 1518550 A | 4-6<br>1-3,7-10 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 November, 2008 (12.11.08) | 25 November, 2008 (25.11.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2008/069335

C (Continuation). DOCUMENTS CONSIDERED TO BE RELEVANT

| Category* | Citation of document, with indication, where appropriate, of the relevant passages | Relevant to claim No. |
|---|---|---|
| X<br>A | GUDMUNDSSON, K.S. et al., Synthesis of Novel Imidazo[1,2-a]pyridines with Potent Activity against Herpesviruses, Organic Letters, 2003, Vol.5/No.8, p.1369-1372 | 4-6<br>1-3,7-10 |
| X<br>A | JP 8-506331 A (Bayer AG.),<br>09 July, 1996 (09.07.96),<br>Full text; particularly, Claims; example Nos.13, 15 on the table 1<br>& US 5658857 A & EP 683779 A1<br>& WO 1994/018198 A1 & DE 4327027 A<br>& AU 6001594 A & BR 9405832 A<br>& CN 1117732 A | 4-6<br>1-3,7-10 |
| X<br>A | JP 63-91391 A (Ortho Pharmaceutical Corp.),<br>22 April, 1988 (22.04.88),<br>Full text; particularly, Claims; example 7<br>& US 4727145 A & EP 261912 A2<br>& AU 7849387 A & DK 495287 A<br>& ZA 8707085 A & DK 164669 B | 4,7<br>1-3,5,6,8-10 |
| X<br>A | ZHUANG, Z. et al., Structure-Activity Relationship of Imidazo[1,2-a]pyridines as Ligands for Detecting beta-Amyloid Plaques in the Brain, Journal of Medicinal Chemistry, 2003, Vol.46/No.2, p.237-243 | 1,2,8,9<br>3-7,10 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007002540 A
- WO 2007063946 A
- JP 2004506723 T
- JP 2005504055 T
- JP 2005512945 T
- JP 2002523383 T
- WO 03106439 A


### Non-patent literature cited in the description


- **J. A. Hardy ; G. A. Higgins.** Alzheimer's Disease: The Amyloid Cascade Hypothesis. *Science,* 1992, vol. 256, 184-185
- **G. McKhann et al.** Clinical diagnosis of Alzheimer's disease: Report of the NINCDS-ADRDA Work Group under the auspices of Department of Health and Human Services Task Force on Alzheimer's Disease. *Neurology,* 1984, vol. 34, 939-944
- **Z.-P. Zhuang et al.** Radioiodinated Styrylbenzenes and Thioflavins as Probes for Amyloid Aggregates. *J. Med. Chem.,* 2001, vol. 44, 1905-1914
- **Masahiro Ono et al.** 11C-labeled stilbene derivatives as Aβ-aggregate-specific PET imaging agents for Alzheimer's disease. *Nuclear Medicine and Biology,* 2003, vol. 30, 565-571
- **H. F. Kung et al.** Novel Stilbenes as Probes for amyloid plaques. *J. American Chemical Society,* 2001, vol. 123, 12740-12741
- **Zhi-Ping Zhuang et al.** IBOX(2-(4'-dimethylaminophenyl)-6-iodobensoxazole): a ligand for imaging amyloid plaques in the braid. *Nuclear Medicine and Biology,* 2001, vol. 28, 887-894
- **Furumoto Y et al.** [11C-]BF-227: A New C-Labeled 2-Ethenylbenzoxazole Derivative for Amylcid-β Plaques Tmaging. *European Journal of Nuclear Medicine and Molecular Imaging,* 2005, vol. 32 (1), 759
- **Eric D. Agdeppa et al.** 2-Dialkylamino-6-Acylmal.ononitrile Substituted Naphtalenes (DDNP Analogs): Novel Diagnostic and Therapeutic Tools in Alzheimer's Disease. *Molecular Imaging and Biology,* 2003, vol. 5, 404-417
- **Zhi-Ping Zhuang et al.** Structure-Activity Relationship of Imidazo[1,2-a]pyridines as Ligands for Detecting β-Amyloid Plaques in the Brain. *J. Med. Chem,* 2003, vol. 46, 237-243
- **W. E. Klunk et al.** Imaging brain amyloid in Alzheimer's disease with Pittsburgh Compound-B. *Ann. Neurol.,* 2004, vol. 55, 306-319
- **Nicolaas P. L. G. Verhoeff et al.** In-Vivo Imaging of Alzheimer Disease β-Amyloid With [11C]SB-13 PET. *American Journal of Geriatric Psychiatry,* 2004, vol. 12, 584-595
- **Hiroyuki Aral et al.** [11C]-BF-227 AND PET to Visualize Amyloid in Alzheimer's Disease Patients. *Alzheimer's & Dementia: The Journal of the Alzheimer's Association,* 2006, vol. 2 (1), S312
- **Christopher M. Clark et al.** Imaging Amyloid with I123 IMPY SPECT. *Alzheimer's & Dementia: The Journal of the Alzheimer's Association,* 2006, vol. 2 (1), 342
- **Zhi-Ping Zhuang et al.** *Nuclear Medicine and Biology,* 2001, vol. 28, 887-894
- **H. F. Kung et al.** *J. Am. Chem. Soc.,* 2001, vol. 123, 12740-12741
- **Masahiro Ono et al.** *Nuclear Medicine and Biology,* 2003, vol. 30, 565-571
- **King, L. Carroll ; Ostrum, G. Kenneth.** *Journal of Organic Chemistry,* 1964, vol. 29 (12), 3459-8461
- *Organic Syntheses,* 2004, vol. 10, 170
- *ORGANIC SYNTHESES,* 2002, vol. 79, 59
- *Organic Syntheses,* 1998, vol. 9, 417
- *ORGANIC SYNTHESES,* vol. 74, 248
- **Zhi-Ping Zhuang et al.** *J. Med. Chem,* 2003, vol. 46, 237-243
- **Douglas D. Dischino et al.** *J. Nucl. Med.,* 1983, vol. 24, 1030-1038